(19) Europäisches Patentamt
European Patent Office
Office européen des brevets

(11) Veröffentlichungsnummer: **0 468 372 A2**

(12) **EUROPÄISCHE PATENTANMELDUNG**

(21) Anmeldenummer: **91112045.9**

(22) Anmeldetag: **18.07.91**

(51) Int. Cl.5: **C07D 471/04**, A61K 31/435, C07D 487/04, C07D 513/04, C07D 233/90, C07D 403/10, C07D 498/04, A61K 31/495, A61K 31/41, //(C07D471/04, 235:00,221:00),(C07D513/04, 277:00,235:00),(C07D513/04, 277:00,221:00),(C07D513/04, 285:00,235:00),(C07D498/04, 271:00,235:00),(C07D487/04, 235:00,231:00)

(30) Priorität: **21.07.90 DE 4023215**

(43) Veröffentlichungstag der Anmeldung:
**29.01.92 Patentblatt 92/05**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Henning, Rainer, Dr.**
**Im Hölchen 16**
**W-6234 Hattersheim am Main(DE)**
Erfinder: **Wagner, Adalbert, Dr.**
**Kleiststrasse 9**
**W-6238 Hofheim am Taunus(DE)**
Erfinder: **Gerhards, Hermann, Dr.**
**Wacholderweg 4**
**W-6238 Hofheim am Taunus(DE)**
Erfinder: **Schölkens, Bernward, Prof. Dr.**
**Hölderlinstrasse 62**
**W-6233 Kelkheim/Taunus(DE)**

(54) **Substituierte Azole, Verfahren zur Herstellung und ihre Verwendung.**

(57) Die Erfindung betrifft Verbindungen der Formel (I),

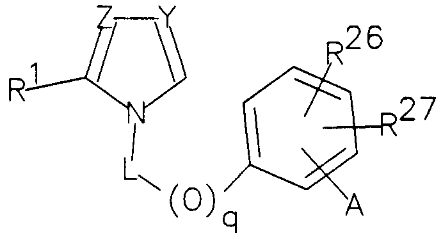

(I)

in welcher
X, Y und Z gleich oder verschieden sind und N oder $CR^2$ bedeuten und die übrigen Reste die in der Beschreibung definierte Bedeutung haben, Verfahren zu deren Herstellung, diese enthaltende Mittel und deren Verwendung.

EP 0 468 372 A2

Aus EP-A-324 377, EP-A-253 310, EP-A-288 833 und EP-A-323 841 sind Derivate von Imidazol, Pyrrol, Pyrazol bzw. Triazol und deren Verwendung als Antagonisten von Angiotensin-II-Rezeptoren bekannt.

Es wurden nun neue Verbindungen vom Azol-Typ gefunden, die hochwirksame Antagonisten von Angiotensin-II-Rezeptoren sowohl in vitro als in vivo sind.

Die Erfindung betrifft Verbindungen der Formel (I)

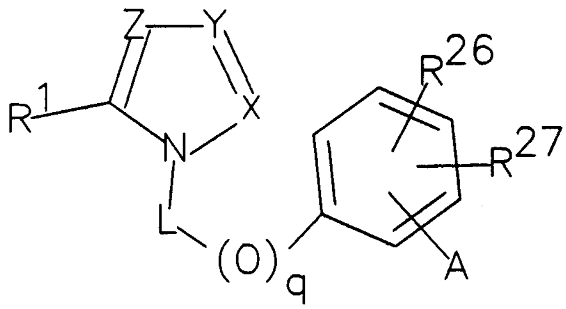

**(I)**

in welcher

a) X, Y und Z gleich oder verschieden sind und N oder $CR^2$ bedeuten,

b)

$R^1$
1. $(C_2-C_{10})$-Alkyl,
2. $(C_3-C_{10})$-Alkenyl,
3. $(C_3-C_{10})$-Alkinyl,
4. $OR^3$,
5. $(C_3-C_8)$-Cycloalkyl,
6. $(C_4-C_{10})$-Cycloalkylalkyl,
7. $(C_5-C_{10})$-Cycloalkylalkenyl,
8. $(C_5-C_{10})$-Cycloalkylalkinyl,
9. $-(CH_2)_m-B-(CH_2)_n-R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1-C_4)$-Alkoxy und Nitro substituiert ist,

bedeutet;

c)

$R^2$
1. Wasserstoff,
2. Halogen,
3. Nitro,
4. $C_vF_{2v+1}$,
5. Pentafluorphenyl,
6. Cyano,
7. Phenyl,
8. Phenyl-$(C_1-C_3)$-alkyl,
9. $(C_1-C_{10})$-Alkyl,
10. $(C_3-C_{10})$-Alkenyl,
11. Phenyl-$(C_2-C_6)$-Alkenyl,
12. 1-Imidazolyl-$(CH_2)_m$-,
13. 1,2,3-Triazolyl-$(CH_2)_n$-,
14. Tetrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,

21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,
25.

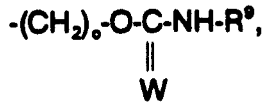

26.

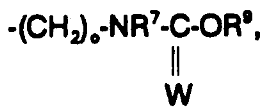

27. $-(CH_2)_o-NR^7-CO-NHR^9$,
28. $-(CH_2)_o-NR^7-SO_2R^9$,
29.

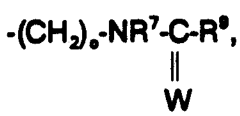

30. $-(CH_2)_nF$,
31. $-(CH_2)_n-O-NO_2$,
32. $-CH_2-N_3$,
33. $-(CH_2)_n-NO_2$,
34. $-CH=N-NR^5R^7$,
35. Phthalimido-$(CH_2)_n$-,
36.

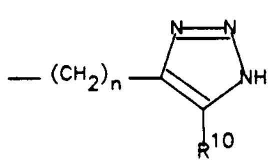

37.

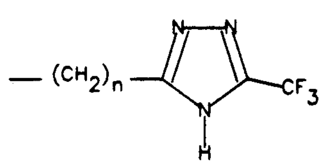

38.

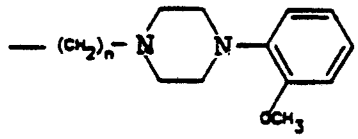

39.

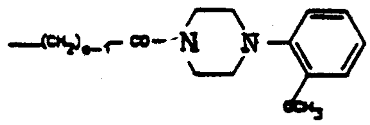

40. Phenyl-$SO_2$-NH-N = CH-,
41.

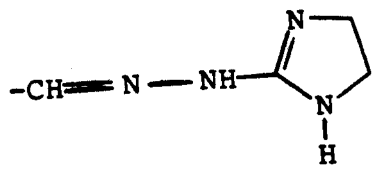

42. -$(CH_2)_n$-$SO_2$-$NR^7$-CO-$NR^6R^9$,
43. -$(CH_2)_o$-$SO_2R^9$,
44. einem wie unter c) 7. oder 8. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,
45. einem wie unter c) 9. oder 10. oder 18. definierten Rest, worin 1 H-Atom durch Hydroxy oder worin 1 bis alle H-Atome durch Fluor substituiert sind, oder
46. den unter c) 13. definierten Rest, der mit 1 oder 2 gleichen oder verschiedenen Resten aus der Reihe Methoxycarbonyl und ($C_1$-$C_4$)-Alkyl substituiert ist,

bedeutet;

d)

$R^3$
1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,

bedeutet;

e)

$R^4$
1. Wasserstoff,
2. ($C_1$-$C_6$)-Alkyl,
3. ($C_3$-$C_8$)-Cycloalkyl, 4. ($C_2$-$C_4$)-Alkenyl oder
5. ($C_2$-$C_4$)-Alkinyl

bedeutet;

f)

$R^5$
1. Wasserstoff,
2. ($C_1$-$C_6$)-Alkyl,
3. ($C_3$-$C_8$)-Cycloalkyl,
4. Phenyl oder
5. Benzyl

bedeutet;

g)

$R^6$
1. Wasserstoff,

2. $(C_1-C_6)$-Alkyl,
3. $(C_3-C_8)$-Cycloalkyl,
4. $(C_6-C_{12})$-Aryl, vorzugsweise Phenyl,
5. Benzyl,
6. $(C_1-C_9)$-Heteroaryl, der teilweise oder vollständig hydriert sein kann, vorzugsweise 2-Pyrimidinyl,
7. $(C_1-C_4)$-Alkanoyl,
8. einen wie unter g) 4. oder 6. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, $NR^{11}R^{12}$ oder

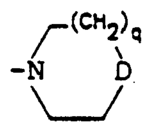

9. $(C_1-C_9)$-Heteroaryl-$(C_1-C_3)$-Alkyl, wobei der Heteroarylteil teilweise oder vollständig hydriert sein kann,

bedeutet;

h)

$R^7$
1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl, vorzugsweise Benzyl,
5. Phenyl oder
6. $(C_1-C_9)$-Heteroaryl

bedeutet;

i)

$R^8$
1. Wasserstoff,
2. $(C_1-C_6)$-Alkyl,
3. $(C_3-C_8)$-Cycloalkyl,
4. Phenyl-$(CH_2)_q$-,
5. $OR^5$,
6. $NR^{11}R^{12}$ oder
7.

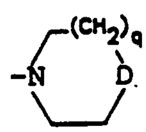

bedeutet;

j)

$R^9$
1. $(C_1-C_6)$-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,

bedeutet;

k)

$R^{10}$ Cyano, Nitro oder $CO_2R^7$ bedeutet;

l)

$R^{11}$ und $R^{12}$ gleich oder verschieden sind und
1. Wasserstoff,

2. $(C_1-C_4)$-Alkyl,
3. Phenyl,
4. Benzyl oder
5. α-Methylbenzyl

bedeuten;

m)

D $NR^{13}$, O oder $CH_2$ bedeutet;

n)

$R^{13}$ Wasserstoff, $(C_1-C_4)$-Alkyl oder Phenyl bedeutet;

o)

A den Rest eines Heterocyclus mit 5-10 Ringatomen, der mono- oder bicyclisch sein kann, und wovon bis zu 9 Ringatome C-Atome sind, der mit bis zu 6, vorzugsweise bis zu 3 gleichen oder verschiedenen Resten $R^{14}$ oder $-(CH_2)_{n-1}-(CHR^6-CH_2)_{o-1}-R^{15}$ substituiert sein kann, und der ungesättigt oder teilhydriert sein kann, bedeutet;

p)

$R^{14}$
1. Halogen,
2. Oxo,
3. Nitroso,
4. Nitro,
5. Amino,
6. Cyano,
7. Hydroxy,
8. $(C_1-C_6)$-Alkyl,
9. $(C_1-C_4)$-Alkanoyl,
10. $(C_1-C_4)$-Alkanoyloxy,
11. $CO_2R^3$,
12. Methansulfonylamino,
13. Trifluormethansulfonylamino,
14. $-CO-NH-OR^9$,
15. $-SO_2-NR^6R^7$,
16. $-CH_2-OR^7$,
17. $(C_1-C_9)$-Heteroaryl-$(CH_2)_q$-, vorzugsweise 1-Tetrazolyl,
18. $(C_7-C_{13})$-Aroyl,
19.

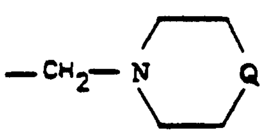

20.

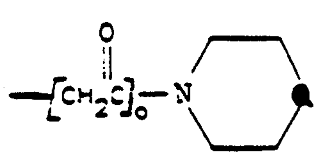

21. $(C_6-C_{12})$-Aryl

bedeutet;

q)

$R^{15}$
1. Wasserstoff,
2. $(C_1-C_6)$-Alkyl,
3. $(C_3-C_8)$-Cycloalkyl,
4. $(C_6-C_{12})$-Aryl,
5. $(C_7-C_{12})$-Aroyl,

6. ($C_1$-$C_4$)-Alkoxy,
7. ($C_1$-$C_4$)-Alkanoyloxy,
8. ($C_1$-$C_9$)-Heteroaryl,
9. $CO_2R^3$,
10. Halogen,
11. Cyano,
12. Nitro,
13. $NR^6R^7$,
14. Hydroxy,
15. -CO-NH-$CHR^5$-$CO_2R^3$,
16. Sulfo,
17. -$SO_3R^3$,
18. -$SO_2$-$NR^7$-CO-$NR^6R^9$,
19. -$NR^7$-CO-$NR^6$-$SO_2$-$CH_2$-$R^5$,
20. -$C(CF_3)_2OH$,
21. Phosphonooxy,
22. -$PO_3H_2$,
23. -NH-$PO(OH)_2$,
24. -$S(O)_rR^6$,
25. -CO-$R^8$,
26. -CO-$NR^6R^9$,
27. -$CR^{20}(OH)$-$PO(OH)_2$,
28. den unter p) 20. definierten Rest,
29.

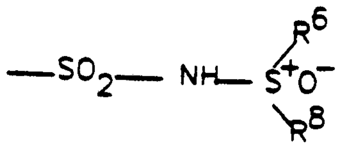

30.

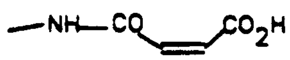

31.

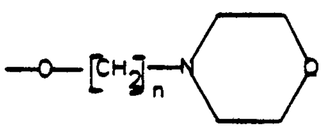

32. 5-Tetrazolyl-NH-CO-,
33. -CO-NH-NH-$SO_2CF_3$,
34.

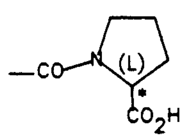

35.

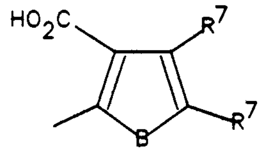

36.

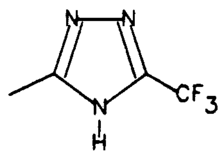

37.

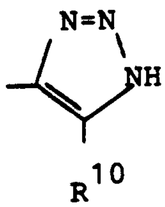

38.

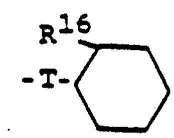

39.

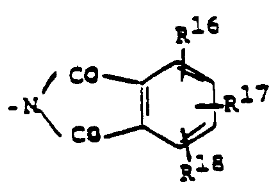

40. $-CO-NH-SO_2-R^{19}$ oder

41. den unter q) 4. definierten Rest, substituiert mit 1 oder 2 gleichen oder verschiedenen Resten aus der Reihe Halogen, Cyano, Nitro, $NR^6R^7$ und Hydroxy bedeutet;

r)
B O, $NR^7$ oder S bedeutet;

s)
W O oder S bedeutet;

t)
L $(C_1-C_3)$-Alkandiyl bedeutet;

u)
$R^{16}$ $CO_2R^3$ oder $CH_2CO_2R^3$ bedeutet;

v)
$R^{17}$ Wasserstoff, Halogen, $(C_1-C_4)$-Alkyl oder $(C_1-C_4)$-Alkoxy bedeutet;

w)

$R^{18}$ Wasserstoff, $(C_1-C_4)$-Alkyl oder Phenyl bedeutet;

x)

$R^{19}$ 1. $(C_1-C_8)$-Alkyl,
2. $(C_3-C_8)$-Cycloalkyl,
3. Phenyl,
4. Benzyl oder
5. den unter x) 1. definierten Rest, worin 1 bis alle H-Atome durch Fluor oder Chlor substituiert sind,

bedeutet;

y)

T 1. eine Einfachbindung,
2. -CO-,
3. $-CH_2-$,
4. -O-,
5. -S-,
6. $NR^{21}$-,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,
15. $SO_2-NR^{21}-$,
16. $-CR^{20}R^{22}-NH-$,
17. -CH = CH-,
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,
25.

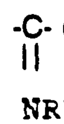 oder

26.

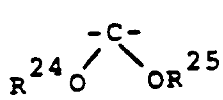

bedeutet;

z)

$R^{20}$ und $R^{22}$ gleich oder verschieden sind und Wasserstoff, $(C_1-C_5)$-Alkyl, Phenyl, Allyl oder Benzyl bedeutet;

a')

$R^{21}$ Wasserstoff, $(C_1-C_6)$-Alkyl, Benzyl oder Allyl bedeutet;

b')

$R^{23}$ 1. $NR^{20}R^{21}$,
2. Ureido,

3. Thioureido,
4. Toluol-4-sulfonyl oder
5. Benzolsulfonylamino

bedeuten;

c')

$R^{24}$ und $R^{25}$ gleich oder verschieden sind und $(C_1-C_4)$-Alkyl bedeuten oder gemeinsam für $-(CH_2)_q-$ stehen;

d')

$R^{28}$ und $R^{27}$ gleich oder verschieden sind und
1. Wasserstoff,
2. Halogen,
3. Nitro,
4. $(C_1-C_4)$-Alkyl oder
5. $(C_1-C_2)$-Alkoxy

bedeuten;

e')

Q $CH_2$, NH, O oder S bedeutet;

f')

m eine ganze Zahl von 0 bis 5 ist;

g')

n eine ganze Zahl von 1 bis 5 ist;

h')

o eine ganze Zahl von 1 bis 10 ist;

i')

q 0 oder 1 ist;

j')

r 0, 1 oder 2 ist, oder

k')

v eine ganze Zahl von 1 bis 6 ist;

sowie deren physiologisch verträglichen Salze. Alkyl, Alkenyl und Alkinyl können geradkettig oder verzweigt sein. Entsprechendes gilt für davon abgeleitete Reste, wie Alkanoyl oder Alkoxy.

Unter Cycloalkyl werden auch alkylsubstituierte Ringe verstanden. $(C_6-C_{12})$-Aryl ist beispielsweise Phenyl, Naphthyl oder Biphenyl, vorzugsweise Phenyl. Entsprechendes gilt für davon abgeleitete Reste, wie Aroyl oder Aralkyl.

Unter $(C_1-C_9)$-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Weiter kann auch 1 oder beide Atome der Kondensationsstelle bicyclischer Reste (wie im Indolzinyl) ein N-Atom sein. Dies sind z.B. Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Unter dem Heterocyclus $AH_2$, von dem der Rest A abgeleitet ist, versteht man beispielsweise Furan, Thiophen, Pyrrol, Imidazol, Pyrazol, Triazol, Oxazol, Isoxazol, Thiazol, Isothiazol, Pyridin, Pyridazin, Pyrimidin, Pyrazin, Indol, Indazol, Chinolin, Isochinolin, Phthalazin, Chinoxalin, Chinazolin, Cinnolin, Benzothiophen, Benzofuran, Cumarin, Chroman, Benzthiazol, Benzoxazol, Benzisothiazol, Benzoxazin, Benzthiazin, Imidazolpyridin, Imidazopyrimidin, Imidazopyrazin, Imidazopyridazin, Imidazotriazin, Imidazothiazol, Imidazoisothiazol, Pyrazolpyridin, Thienopyridin, Furopyridin, Oxazolopyridin, Oxazolopyrimidin und Pyrrolopyrimidin. Falls der Heterocyclus teilhydriert ist, bleibt vorzugsweise ein Rest aromatisch.

Die Verknüpfung von A erfolgt vom isocyclischen oder vom heterocyclischen Teil aus über eine Alkandiyl-Brücke L.

Unter physiologisch verträglichen Salzen von Verbindungen der Formel I versteht man sowohl deren organische als auch anorganische Salze, wie sie in Remington's Pharmaceutical Sciences, 17. Auflage, Seite 1418 (1985) beschrieben sind. Aufgrund von physikalischer und chemischer Stabilität und Löslichkeit sind für saure Gruppen unter anderem Natrium-, Kalium-, Calcium- und Ammoniumsalze bevorzugt; für basische Gruppen unter anderen Salze mit Salzsäure, Schwefelsäure, Phosphorsäure, Carbonsäuren oder Sulfonsäuren, wie Essigsäure, Citronensäure, Benzoesäure, Maleinsäure, Fumarsäure, Weinsäure, p-Toluolsulfonsäure.

Bevorzugt sind Verbindungen der Formel I, worin

a) X N, Y $CR^2$ und Z $CR^2$ bedeuten;
b) X $CR^2$, Y N und Z $CR^2$ bedeuten;
c) X $CR^2$, Y $CR^2$ und Z N bedeuten
oder
d) X, Y und Z jeweils N bedeuten, wobei c) besonders bevorzugt ist.

Weiter sind Verbindungen der Formel (I) bevorzugt, in welcher

a)
$R^1$
1. $(C_3$-$C_{10})$-Alkyl,
2. $(C_3$-$C_{10})$-Alkenyl,
3. $(C_3$-$C_{10})$-Alkinyl,
4. $(C_3$-$C_8)$-Cycloalkyl,
5. Benzyl oder
6. Benzyl, das wie oben beschrieben substituiert ist,

bedeutet;

b)
$R^2$
1. Wasserstoff,
2. Halogen,
3. Nitro,
4. $C_v F_{2v+1}$,
5. Pentafluorphenyl,
6. Cyano,
7. Phenyl,
8. Phenyl-$(C_1$-$C_3)$-alkyl,
9. $(C_1$-$C_{10})$-Alkyl,
10. $(C_3$-$C_{10})$-Alkenyl,
11. Phenyl-$(C_2$-$C_6)$-alkenyl,
12. 1-Imidazolyl-$(CH_2)_m$-,
13. 1,2,3-Triazolyl-$(CH_2)_o$-,
14. Tetrazolyl-$(CH_2)_m$-,
15. $-(CH_2)_{0-1}$-$CHR^7$-$OR^5$,
16. $-(CH_2)_o$-O-$COR^3$,
17. $-COR^8$,
18. $-(CH_2)_o$-CO-$R^8$,
19. $-S(O)_rR^6$,
20. $-CH=CH-(CH_2)_m$-$CHR^3$-$OR^{6'}$,
21. $-CH_2=CH-(CH_2)_m$-CO-$R^8$,
22. $-(CH_2)_o$-NH-CO-$OR^9$,
23. $-(CH_2)_o$-NH-$SO_2$-$R^9$,
24. $-(CH_2)_nF$,
25. $-(CH_2)_o$-$SO_3R^9$,
26. $-(CH_2)_n$-$SO_2$-NH-CO-$NR^6R^9$ oder
27. einem wie unter b) 7., 8., 9., 10. oder 15. definierten Rest, der wie oben unter c) 44., 45. oder 46. jeweils für einen solchen Rest beschrieben substituiert ist,

bedeutet;

c)
$R^8$ Wasserstoff; $(C_1$-$C_5)$-Alkyl, $OR^5$, $NR^{11}R^{12}$ oder Morpholino

bedeutet;

d)
T
1. eine Einfachbindung,
2. -CO-,
3. $-CONR^{21}$-,
4. $-CH_2$-$CH_2$-,
5. $-NR^{21}$-CO-,
6. -O-$CH_2$-,
7. $-CH_2$-O-,
8. -S-$CH_2$-,
9. $-CH_2$-S-,
10. -NH-$CH_2$-,

11. $-CH_2-NH-$ oder
12. $-CH=CH-$

bedeutet, und die übrigen Reste und Variablen wie oben definiert sind.

Besonders bevorzugt sind Verbindungen der Formel (I); in welcher

a)
$R^1$ $(C_3-C_7)$-Alkyl, $(C_3-C_7)$-Alkenyl oder $(C_3-C_7)$-Alkinyl bedeutet;

b)
$R^2$ 1. Chlor,
2. Brom,
3. $C_v F_{2v+1}$ mit v = 1, 2 oder 3,
4. Pentafluorphenyl,
5. $-S(O),R^6$,
6. $(CH_2)_{0-1}-CHR^7-OR^5$,
7. $(CH_2)_o-O-CO-R^3$,
8. $-COR^8$,
9. $-(CH_2)_o-CO-R^8$,
10. $-CH_2-NH-CO-R^8$,
11. $-(CH_2)_o-NH-SO_2-R^9$,
12. $-CH=CH-CHR^3-OR^6$,
13. Tetrazolyl-$(CH_2)_m$-,
14. $-(CH_2)_nSO_2-NH-CO-NR^6R^9$,
15. $-(CH_2)_o-SO_3R^9$ oder

gegebenenfalls durch Hydroxy substituiertes $(C_1-C_6)$-Alkyl, vorzugsweise Hydroxymethyl, bedeutet;

c)
$R^3$ Wasserstoff oder $(C_1-C_4)$-Alkyl bedeutet;

d)
$R^6$ Wasserstoff, $(C_1-C_4)$-Alkyl, $(C_1-C_4)$-Alkanoyl oder vorzugsweise $(C_2-C_9)$-Heteroaryl bedeutet;

e)
$R^7$ Wasserstoff, $(C_1-C_4)$-Alkyl, $(C_1-C_9)$-Heteroaryl, oder $(C_6-C_{12})$-Aryl-$(C_1-C_4)$-Alkyl bedeutet;

f)
$R^8$ Wasserstoff, $(C_1-C_4)$-Alkyl, $OR^5$ oder Morpholino bedeutet;

g)
$R^9$ $CF_3$, $(C_1-C_6)$-Alkyl oder Phenyl bedeutet;

h)
$R^{14}$ 1. $(C_1-C_4)$-Alkyl,
2. $(C_1-C_4)$-Alkoxy,
3. Cyano,
4. Amino,
5. Nitroso,
6. Nitro,
7. Fluor,
8. Chlor,
9. Brom,
10. Hydroxy,
11. $CH_2OR^7$,
12. $(C_1-C_9)$-Heteroaryl-$CH_2$-,
13. $(C_1-C_4)$-Alkanoyloxy,
14. $(C_1-C_4)$-Alkanoyl,
15. Benzoyl,
16.

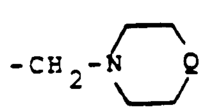

17. -NH-CO-$R^7$ oder
18. Tetrazolyl

bedeutet;

i)

$R^{15}$
1. $(C_1-C_4)$-Alkyl,
2. $(C_6-C_{12})$-Aryl,
3. $(C_1-C_3)$-Alkanoyloxy,
4. $(C_1-C_4)$-Alkoxy,
5. $(C_1-C_9)$-Heteroaryl, vorzugsweise 5-Tetrazolyl,
6. Cyano,
7. Nitro,
8. Hydroxy,
9. $-S(O)_rR^6$,
10. $-SO_3R^3$,
11. Chlor,
12. Brom,
13. Benzoyl,
14. $-CO_2R^3$,
15. -CO-NH-$R^6$,
16. $-NR^6R^7$,
17. -CO-$R^8$,
18. $-SO_2-NR^6R^7$,
19.

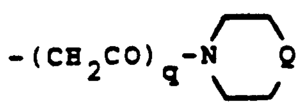

20.

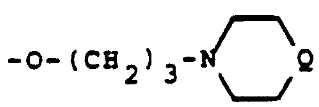

21. $-SO_2$-NH-CO-$NR^6R^9$,
22. $-PO_3H_2$,
23. -CO-$CHR^5$-$CO_2H$,
24. -NH-CO-NH-$SO_2$-$CH_2$-$R^5$,
25. 5-Tetrazolyl-NH-CO-,
26.

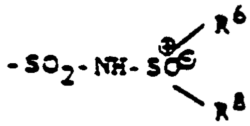

27.

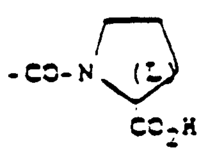

28.

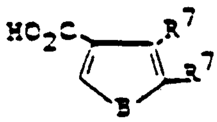

29.

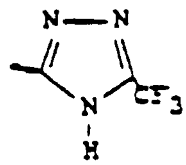

30.

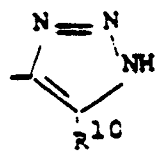

31.

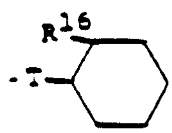

32.

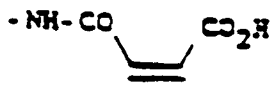

33. $-CO-NH-SO_2R^{19}$ oder
34. den unter i) 2. definierten Rest, substituiert wie oben definiert,

bedeutet;

j)
Q $CH_2$, NH oder O bedeutet;

k)
$R^{18}$ Wasserstoff, Methyl oder Ethyl bedeutet;

l)
T eine Einfachbindung, -O-, -CO-, -NHCO- oder $-OCH_2-$ bedeutet,

und die übrigen Reste und Variablen wie oben definiert sind.

Ganz besonders bevorzugt sind Verbindungen der Formel (I), wobei die Symbole $R^2$, $R^9$, $R^{14}$, $R^{15}$, Z, X, Y, q folgende Bedeutung haben:

$R^2$ Chlor, Brom, $-S(O)_rR^6$ oder $-COR^8$;
$R^9$ $(C_1-C_6)$-Alkyl;
$R^{14}$ Tetrazolyl;

$R^{15}$ $-CO_2-R^3$, $SO_2-NR^6R^7$, $-SO_2-NH-CO-NR^6R^9$ oder $-NH-CO-NH-SO_2-CH_2-R^5$;
Z gleich N;
X, Y beide $CR^2$ bedeuten;
q Null;
L $CH_2$;

Die Erfindung betrifft auch ein Verfahren zur Herstellung von Verbindungen der Formel I, das dadurch gekennzeichnet ist, daß man Verbindungen der Formel (II)

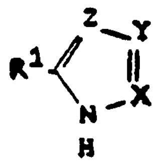

(II)

worin $R^1$, X, Y und Z wie oben definiert sind, alkyliert mit Verbindungen der Formel III

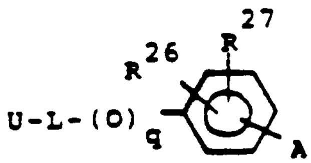

(III)

worin L, A und q wie oben definiert sind und U für eine Fluchtgruppe steht, gegebenenfalls temporär eingeführte Schutzgruppen wieder abspaltet und die erhaltenen Verbindungen der Formel (I) gegebenenfalls in ihre physiologisch verträglichen Salze überführt.

Geeignete Fluchtgruppen U sind vorzugsweise nucleofuge Gruppen (vgl. Angew. Chem. 72 (1960) 71) wie Halogen, o-Toluolsulfonat, Mesylat oder Triflat.

Verfahren zur Herstellung der Vorstufen der Formel (II) sind u.a. bekannt aus US-4 355 044, EP-A-324 377 und EP-A-323 841.

Weitere Verfahren sind beschrieben in G. L'abbe (Chem. Rev. 69, 345 (1969)); T. Srodsky (in "The Chemistry of the Azido Group", Wiley, New York, 1971, S. 331); H. Wamhoff (in "Comprehensive Heterocyclic Chemistry", A. Katritzky Ed., Pergamon Press, New York (1984)). Ein weiteres Verfahren geht von 1-Cyanoglyoxylsäure-2-oxim-Derivaten aus und liefert nach Reduktion des Oxims mit literaturbekannten Reduktionsmitteln und Addition von Mercaptoverbindungen an die Nitrilgruppe unter Verwendung geeigneter Schutzgruppen Vorstufen, die unter wasserabspaltenden Bedingungen zu Imidazolen cyclisiert werden können. Für den Cyclisierungsschritt können u.a. Gemische aus $PCl_5$ und Dimethylaminopyridin (DMAP), $POCl_3$ und $SOCl_2$ und deren Gemische mit DMAP verwendet werden.

Die Oxidation der Thioverbindungen zu den entsprechenden Sulfonen und Sulfoxiden erfolgt bevorzugt durch Persäuren in geeigneten Lösungsmitteln wie z.B. Dichlormethan.

Zur Alkylierung der Azole der Formel (II) sind z.B. entsprechende Benzylhalogenide, - tosylate, -mesylate oder -triflate oder entsprechende Alkylhalogenide, -tosylate, -mesylate oder -triflate geeignet.

Die Darstellung dieser Verbindungen erfolgt in an sich bekannter Weise, beispielsweise durch Halogenierung der entsprechenden Methylvorstufen. Hierzu wird vorzugsweise N-Bromsuccinimid eingesetzt, siehe z.B. J. Org. Chem. 44, 4733 (1979) und Helv. Chim. Acta 62, 2661 (1979).

Die Alkylierung erfolgt nach prinzipiell bekannten Verfahren in analoger Weise.

Das Azol-Derivat der Formel (II) wird z.B. in Gegenwart einer Base metalliert. Bevorzugte Basen sind Metallhydride der Formel MH wie z.B. Lithium-, Natrium- oder Kaliumhydrid in beispielsweise DMF oder DMSO als Lösungsmittel oder Metallalkoxide der Formel MOR, wobei R für Methyl, Ethyl, t-Butyl steht und die Reaktion in dem entsprechenden Alkohol, DMF oder DMSO durchgeführt wird. Die so gebildeten Salze der Azole werden in einem aprotischen Lösungsmittel wie DMF oder DMSO gelöst und mit einer geeigneten Menge Alkylierungsreagenz versetzt.

Eine alternative Möglichkeit zur Deprotonierung der Azol-Derivate stellt z.B. die Umsetzung mit Kaliumcarbonat in DMF oder DMSO dar.

Die Herstellung der Tetrazole erfolgt nach prinzipiell bekannten Methoden aus den entsprechenden Nitrilen mit Aziden wie z. B. Trialkylzinnaziden oder Natriumazid.

Die Umsetzungen werden bei Temperaturen unterhalb Raumtemperatur bis zum Siedepunkt des

Reaktionsgemisches, vorzugsweise zwischen +20 °C und dem Siedepunkt des Reaktionsgemisches während etwa 1 bis 10 Stunden durchgeführt.

Die erfindungsgemäßen Verbindungen der Formel I haben antagonistische Wirkung auf Angiotensin-II-Rezeptoren und können deshalb zur Behandlung der Angiotensin-II-abhängige Hypertension verwendet werden. Anwendungsmöglichkeiten bestehen weiterhin bei Herzinsuffizienz, Kardioprotektion, Myocardinfarkt, Herz-Hypertrophie, Arteriosklerose, Nephropathie, Nierenversagen sowie vasculären Erkrankungen des Gehirn wie transitorischen ischämischen Attacken und Gehirnschlag.

Renin ist ein proteolytisches Enzym aus der Klasse der Aspartyl-proteasen, welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt.

Postrezeptor-Wirkungen sind unter anderen Stimulation des Phosphoinositol-Umsatzes ($Ca^{2+}$-Freisetzung), Aktivierung der Proteinkinase C und Facilitation von cAMP-abhängigen Hormon-Rezeptoren.

Die Affinität der Verbindungen der Formel I zum Angiotensin-II-Rezeptor kann durch Messung der $^{125}$I-Angiotensin-II- oder $^{3}$H-Angiotensin-II-Verdrängung von Rezeptoren an Zona glomerulosa Membranen von Rindernebennieren bestimmt werden. Dazu werden die präparierten Membranen in Puffer bei pH 7,4 suspendiert. Um die Degradierung des Radioliganden während der Inkubierung zu verhindern, wird Aprotinin, ein Peptidase Inhibitor, zugesetzt. Zusätzlich werden ca. 14000 cpm eines Tracers mit spezifischer Aktivität von 74f TBq/mmol (käuflich bei Amersham Buchler) und eine Menge Rezeptorprotein, die 50 % des Tracers bindet, verwendet. Die Reaktion wird durch Zugabe von 50 µl Membransuspension zu einer Mischung von 100 µl Puffer + Aprotinin; 50 µl Puffer mit oder ohne Angiotensin-II oder Rezeptorantagonist und 50 µl Tracer gestartet. Nach einer Inkubationszeit von 60 Minuten bei 25 °C werden gebundener und freier Radioligand durch einen Filtrationsassay mit Whatman® GFIC Filtern auf einem Skatron®-Zellsammler getrennt.

Unspezifische Bindungen werden durch Behandlung der Filter mit 0,3 % Polyethylenimin pH = 10 verhindert (Sigma, Nr. 3143). Durch Messung der Radioaktivität in einem Gamma-Szintillationszähler wird die Stärke der Verdrängung des Radioliganden vom Rezeptor bestimmt. Die $IC_{50}$-Werte, welche die Konzentration des Inhibitors bedeutet, um 50 % des Liganden zu verdrängen, werden nach Chem. et. al. J. Theor. Biol. 59, 253 (1970) bestimmt. Sie liegen für die Verbindungen der Formel (I) im Bereich von $1x10^{-4}$ - $1x10^{-9}$ M.

Zur Bestimmung der antagonistischen Wirkung der Verbindungen der Formel (I) kann ihr Effekt auf den Angiotensin-II-induzierten Blutdruckanstieg an narkotisierten Sprague-Dawley Ratten gemessen werden. Als Narkotikum dient Na-Thiobarbital (Trapanal®, Byk Gulden) in der Dosierung 100 mg/kg i.p. Die i.v.-Applikation erfolgen in die Vena Jugularis. Der Blutdruck wird in der A. carotis gemessen. Zunächst werden die Tiere mit Pentoliniumtartrat (10 mg/kg im.) vorbehandelt, so daß ein niedrigerer Blutdrucklevel erreicht wird (Ganglienblockade). ANG II (Hypertensin (CIBA)) wird im Volumen 0,1 ml/100 g in 10-minütigen Intervallen i.v. appliziert. Die Dosis beträgt 0,5 µg/kg. Die Verbindungen der Formel (I) werden in Aqua dest. gelöst und in den Dosierungen 0,1 bis 1,10 und 100 mg/kg intravenös oder intraduodenal appliziert.

Die Verbindungen der Formel (I) sind insbesondere im Bereich von 0,1-100 mg/kg wirksam.

Die Erfindung bezieht sich ebenso auf pharmazeutische Zubereitungen bestehend aus einer Verbindung der Formel (I) und anderen Wirkstoffen, wie z.B. Diuretika oder nichtsteroidaler anti-inflammatorischen Wirkstoffen. Die Verbindungen der Formel (I) können auch als Diagnostika für das Renin-Angiotensin-System verwendet werden.

Pharmazeutische Präparate enthalten eine wirksame Menge des Wirkstoffs der Formel (I) und eventuell andere Wirkstoffe zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, dem Alter und von der Applikationsart ab.

Die pharmazeutischen Präparate der vorliegenden Erfindung werden in an sich bekannten Lösungs-, Misch-, Granulier- oder Dragierverfahren hergestellt. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen wie Tabletten, Dragees, Steckkapseln, wäßrige, alkoholische oder ölige Suspensionen oder wäßrige, alkoholische oder ölige Lösungen gebracht. Als inerte Träger können z.B. Gummi arabicum, Magnesia, Magnesiumcarbonat,

Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittlern, Emulgatoren oder weiteren Hiffsstoffen in Lösungen, Suspensionen oder Emulsionen gebracht. Als Lösungsmittel kommen z.B. in Frage: Wasser, physiologische Kochsalzlösung oder Alkohole, z.B. Ethanol, Propandiol oder Glycerin, daneben auch Zuckerlösungen wie Glucose- oder Mannitlösungen oder auch eine Mischung aus den verschiedenen genannten Lösungsmitteln.

Liste der Abkürzungen:

| | |
|---|---|
| DMF | N,N-Dimethylformamid |
| NBS | N-Bromsuccinimid |
| AIBN | $\alpha,\alpha$-Azobis-isobutyronitril |
| EI | electron impact |
| DCI | Desorption Chemische Ionisation |
| RT | Raumtemperatur |
| EE | Ethylacetat |
| DIP | Diisopropylether |

**Beispiel 1**

2-[4-[(2-n-Butyl-4-chlor-5-formyl-imidazol-1-yl)methyl]phenyl]imidazo[1,2-a]pyridin-3-carbonsäure

a) 2-Brom-3-p-tolyl-3-oxo-propionsäure-ethylester
20,4 g 3-p-Tolyl-3-oxo-propionsäure-ethylester (Helv. Chim. Acta 57, 2205 (1974)) werden in 20 ml $CCl_4$ gelöst. Eine Lösung von 6 ml Brom in 30 ml $CCl_4$ wird bei -5 °C zugetropft. Nach einer Stunde bei -5 °C wird 3 Std. bei 20 °C, dann 1 Std. bei 60 °C gerührt. Das Lösungsmittel wird entfernt. Die Titelverbindung wird als Rohprodukt weiterverwendet; Ausbeute 34 g.

b) 2-(4-Methyl-phenyl)-imidazo[1,2-a]pyridin-3-carbonsäure-ethylester
5,7 g (20 mmol) der Verbindung aus 1a) und 3,76 g (40 mmol) 2-Aminopyridin werden in 50 ml absolutem EtOH 4 Std. am Rückfluß gekocht, dann über Nacht bei RT gerührt. Nach Einengen wird in 1 N $NaHCO_3$-Lösung aufgenommen und 3x mit $CH_2Cl_2$ extrahiert. Nach Trocknen über $Na_2SO_4$ wird eingeengt. Das Rohprodukt wird an $SiO_2$ mit EtOAc/n-Heptan (1:2) chromatographiert. Durch Kristallisation aus n-Heptan erhält man 4,1 g Produkt vom Schmp. 88 °C; MS (DCI) = 281 (M + H)

c) 2-(4-Brommethyl-phenyl)-imidazo[1,2a]pyridin-3-carbonsäure-ethylester
3 g (10,7 mmol) der Verbindung aus 1b) werden in 20 ml $CCl_4$ mit 2,1 g (11,8 mmol) NBS und 200 mg Benzoylperoxid 4 Std. am Rückfluß gekocht. Nach Abkühlen wird abgesaugt und das Filtrat mit $NaHCO_3$ (1N) 2x extrahiert. Die organische Phase wird über $Na_2SO_4$ getrocknet und eingeengt. Chromatographie an Kieselgel mit EtoAc/n-Heptan (0,8:1,2) als Laufmittel ergibt 1,5 g der Titelverbindung als farblose Kristalle; Schmp. 131 °C MS (DCI): 359 + 361 (M + H)

d) 2-[4-[(2-n-Butyl-4-chlor-5-formyl-imidazol-1-yl)methyl]phenyl]imidazo[1,2-a]pyridin-3-carbonsäure-ethylester (A)
0,72 g (2 mmol) der Verbindung aus 1c), 0,37 g (2 mmol) 2-n-Butyl-4-chlorimidazol-5-aldehyd (aus EP-A-324 377) sowie 0,3 g (2,2 mmol) Kaliumcarbonat werden in 10 ml trockenen DMF 3 Std. bei RT gerührt. Nach Aufnehmen in Wasser wird mit EtOAc extrahiert (2x). Die vereinigten organischen Phasen werden 3 mal mit $H_2O$ und 1 mal mit gesättigter NaCl-Lösung gewaschen, mit $Na_2SO_4$ getrocknet und eingeengt. Chromatographie an Kieselgel liefert 0,8 g der Titelverbindung sowie 0,04 g des 5-Chlor-4-formyl-Isomeren B.

A: $^1$H-NMR (270 MHz, $CDCl_3$): $\delta$ = 9,78 (s, 1H); 9,39 (d, 1H); 7,74 (d, 2H); 7,70 (d, 1H); 7,43 (dt, 1H); 7,09 (d, 2H); 7,03 (dt, 1H); 5,63 (s, 1H); 4,32 (q, 2H); 2,67 (m, 2H); 1,7 (m, 2H); 1,4 (m, 2H); 1,22 (t, 3H); 0,9 (t, 3H) ppm
$R_f$ ($SiO_2$; EtOAc/n-Heptan (1:2) = 0,16

B: $^1$H-NMR (270 MHz, $CDCl_3$: $\delta$ = 9,93 (s, 1H); 9,39 (d, 1H); 7,78 (d, 2H); 7,72 (m, 1H); 7,46 (dt, 1H); 7,08 (d, 2H); 7,02 (dt, 1H); 5,76 (s, 2H); 4,31 (q, 2H); 2,68 (m, 2H); 1,75 (m, 2H); 1,4 (m, 2H); 1,25 (t, 3H); 0,9 (t, 3H) ppm
$R_f$ ($SiO_2$; EtOAc/n-Heptan (1:1)) = 0,08

e) 2-[4-[(2-n-Butyl-4-chlor-5-formyl-imidazol-1-yl)methyl]phenyl]imidazo[1,2-a]pyridin-3-carbonsäure

0,28 g (0,6 mmol) des Isomers A aus Beispiel 1d) werden in 5 ml Ethanol mit 1,2 ml 1 N NaOH 18 Std. bei RT gerührt (unter Stickstoff). Nach Verdünnen mit 10 %iger $KH_2PO_4$-Lösung wird mit EtOAc 3x extrahiert. Nach Waschen mit gesättigter NaCl-Lösung wird mit $Na_2SO_4$ getrocknet und eingeengt. Das Rohprodukt wird aus Isopropylester kristallisiert. Man erhält 0,16 g der Titelverbindung als farblose Kristalle, Schmp. 120-123 °C MS (DCI): 437 (M + H)

**Beispiel 2**

2-[4-[(2-n-Butyl-4-chlor-5-hydroxymethyl-imidazol-1-yl)methyl]phenyl]-imidazo[1,2-a]pyridin-3-carbonsäure

a) 2-[4-(2-n-Butyl-4-chlor-5-hydroxymethyl-imidazol-1-yl]methyl]phenyl]imidazo[1,2-a]pyridin-3-carbonsäure-ethylester

0,28 g der Verbindung A aus Beispiel 1d) werden in 10 ml Ethanol mit 0,25 g Natriumborhydrid 45 min gerührt. Nach Verdünnen mit 1 N NaOH wird 2x mit EtOAc extrahiert. Nach Waschen der organischen Phase mit ges. NaCl-Lösung wird mit $Na_2SO_4$ getrocknet und eingeengt. Man erhält 0,22 g der Titelverbindung.

$^1$H-NMR (270 MHz, $CDCl_3$): $\delta$ = 9,4 (dt, 1H); 7,75 (d, 2H); 7,73 (dt, 1H); 7,46 (dt, 1H); 7,05 (m, 3H); 5,3 (s, 2H); 4,5 (s, 2H); 4,3 (q, 2H); 2,6 (m, 2H); 1,7 (m, 2H); 1,48 (m, 2H); 1,25 (t, 3H); 0,9 (t, 3H) ppm

MS (FAB): 467 (M + H)

b) 2-[4-[(2-n-Butyl-4-chlor-5-hydroxymethyl-imidazol-1-yl]methyl]phenyl]imidazo[1,2-a]pyridin-3-carbonsäure

0,22 g der Verbindung aus Beispiel 2a) werden in 5 ml Ethanol mit 0,9 ml 1N NaOH entsprechend Beispiel 1e) umgesetzt. Man erhält 0,14 g der Titelverbindung als farblose Kristalle vom Schmp. 173-175 °C MS (FAB): 439 (M + H)

**Beispiel 3**

2-[4-[(2-n-Butyl-5-carboxy-4-chlor-imidazol-1-yl]methyl]phenyl]imidazo[1,2-a]pyridin-3-carbonsäure

a) 2-[4-[(2-n-Butyl-4-chlor-5-ethoxycarbonyl-imidazol-1-yl]methyl]phenyl]imidazo[1,2-a]pyridin-3-carbonsäure-ethylester

0,28 g (0,6 mmol) der Verbindung A aus Beispiel 1d) werden in 5 ml Ethanol gelöst. Man gibt 0,15 g Natriumcyanid zu, gefolgt von 53 µl Eisessig und 1,25 g Mangandioxid. Nach 32 Std. Rühren bei RT wird abgesaugt, mit Ethanol gewaschen und das Filtrat eingeengt. Nach Aufnehmen in $H_2O$ wird mit 2 % HCl auf PH 3-4 gestellt und mit $CH_2Cl_2$ extrahiert. Nach Trocknen der organischen Phase mit $Na_2SO_4$ wird eingeengt. Das Rohprodukt wird ohne weitere Reinigung umgesetzt.

b) 2-[4-(2-n-Butyl-5-carboxy-4-chlor-imidazol-1-yl]methyl]phenyl]imidazo[1,2-a]pyridin-3-carbonsäure

Das Rohprodukt aus 3a) wird mit 2 ml 1 N NaOH in 3 ml Ethanol 48 Std. bei RT gerührt. Nach Einengen wird in Wasser aufgenommen und mit 2 % HCl auf PH 3 gestellt. Nach Sättigen mit NaCl wird mit $CH_2Cl_2$ extrahiert, mit $Na_2SO_4$ getrocknet und eingeengt. Das Rohprodukt wird an Kieselgel mit $CH_2Cl_2$/MeOH (2:1) gereinigt. Man erhält 40 mg der

Titelverbindung.

MS (FAB): 453 (M + H)

**Beispiel 4**

2-[4-[(2-n-Butyl-4-chlor-5-formyl-imidazol-1-yl)methyl]phenyl]imidazo[1,2-a]pyrimidin-3-carbonsäure

a) 2-(4-Methyl-phenyl)-imidazo[1,2-a]pyrimidin-3-carbonsäure-ethylester

5,7 g der Verbindung aus 1a) werden mit 10 g 2-Aminopyrimidin 30 min auf 130 °C erwärmt. Nach Abkühlen wird in $CH_2Cl_2$ aufgenommen und 6x mit $H_2O$ gewaschen. Nach Trocknen über $Na_2SO_4$ wird eingeengt. Das Rohprodukt wird an Kieselgel mit EtoAc als Laufmittel gereinigt. Man erhält 3,45 g der Titelverbindung als farblose Kristalle. MS (DCI): 282 (M + H)

b) 2-(4-Brommethyl-phenyl)imidazo[1,2-a]pyrimidin-3-carbonsäure-ethylester

3,3 g der Verbindung aus 4a) werden mit 2,4 g NBS und 230 mg Benzoylperoxid in 35 ml $CCl_4$ 4 Std. am Rückfluß gekocht. Die Aufarbeitung erfolgte nach den in Beispiel 1c) angegebenen Verfahren. MS

(DCI): 360 + 362 (M + H)

c) 2-[4-[(2-n-Butyl-4-chlor-5-formyl-imidazol-1-yl)methyl]phenyl]imidazo[1,2-a]pyrimidin-3-carbonsäure-ethylester

Hergestellt nach dem in Beispiel 1d) angegebenen Verfahren aus der Verbindung aus Beispiel 4b).

MS (FAB) 466 (M + H)

d) 2-[4-[(2-n-Butyl-4-chlor-5-formyl-imidazol-1-yl)methyl]phenyl]imidazo[1,2-a]pyrimidin-3-carbonsäure

Hergestellt nach dem in Beispiel 1e) angegebenen Verfahren aus der Verbindung von Beispiel 4c).

MS (FAB) 438 (M + H)

Die in der folgenden Tabelle aufgeführten Beispiele der Formel Ia wurden analog den in den Beispielen 1-4 angegebenen Vorschriften hergestellt.

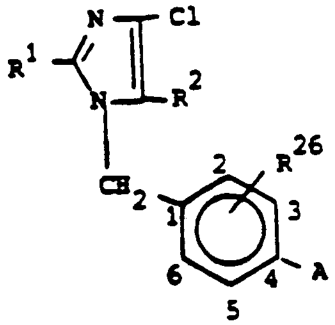

| Beispiel | $R^1$ | $R^2$ | $R^{26}$ | A |
|---|---|---|---|---|
| 5 | n-$C_4H_9$ | $CH_2OH$ | H | 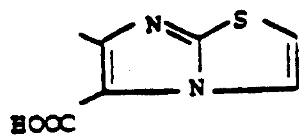 |
| 6 | n-$C_3H_7$ | CHO | 2-Cl | 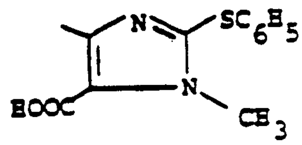 |
| 7 | n-$C_4H_9$ | $CH_2OH$ | H | 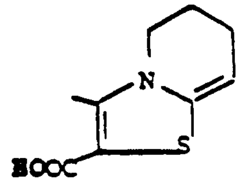 |
| 8 | $CH_3CH_2CH=CH$ | $CH_2OH$ | H | 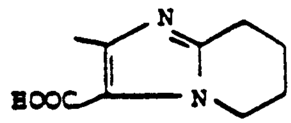 |
| 9 | n-$C_4H_9$ | CHO | H | 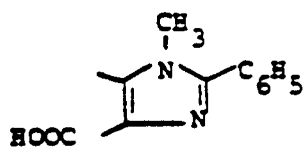 |
| 10 | n-$C_4H_9$ | $CH_2OH$ | 2-$OCH_3$ | 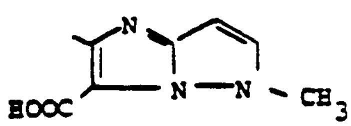 |

| | | | | |
|---|---|---|---|---|
| 11 | n-$C_5H_{11}$ | CHO | H | 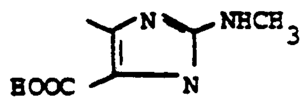 |
| 12 | n-$C_4H_9$ | $CH_2OH$ | H | 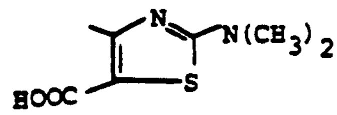 |
| 13 | n-$C_4H_9$ | COOH | H | 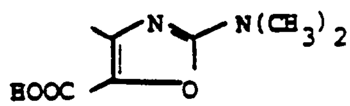 |
| 14 | n-$C_4H_9$ | $CH_2OH$ | H | 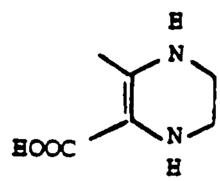 |
| 15 | n-$C_4H_9$ | CHO | H | 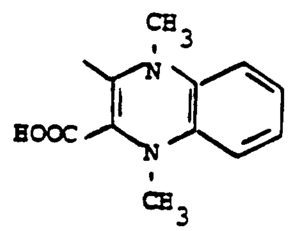 |
| 16 | n-$C_3H_7$ | $CH_2OH$ | H | 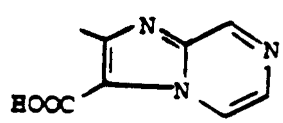 |
| 17 | n-$C_4H_9$ | CHO | H | 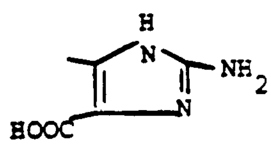 |
| 18 | n-$C_4H_9$ | $CH_2OH$ | 2-$OCH_3$ | 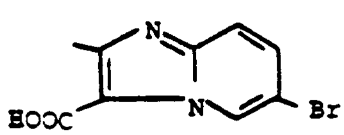 |

| | | | | |
|---|---|---|---|---|
| 19 | n-$C_4H_9$ | CHO | H | 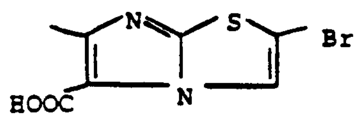 |
| 20 | n-$C_3H_7$ | CHO | H | 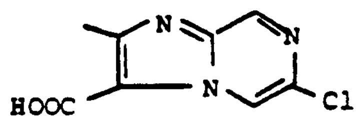 |
| 21 | n-$C_4H_9$ | $CH_2OH$ | H | 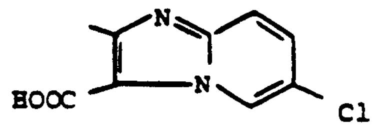 |
| 22 | n-$C_3H_7$ | CHO | H | 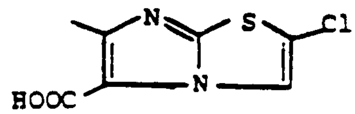 |
| 23 | n-$C_4H_9$ | $CH_2OH$ | H | 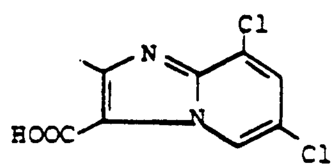 |
| 24 | n-$C_4H_9$ | $CH_2OH$ | H | 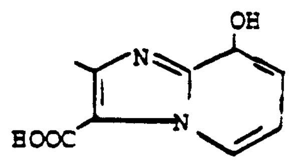 |
| 25 | n-$C_4H_9$ | $CH_2OH$ | H | 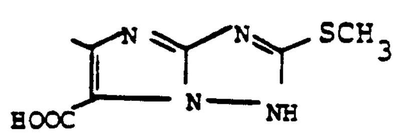 |
| 26 | n-$C_4H_9$ | $CH_2OH$ | H | 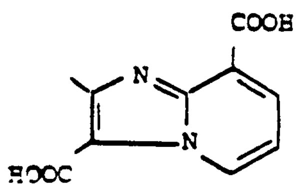 |

| | | | | |
|---|---|---|---|---|
| **27** | n-$C_4H_9$ | CHO | 2-Cl | 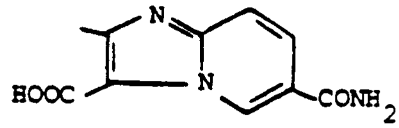 |
| **28** | n-$C_4H_9$ | CHOH | H | 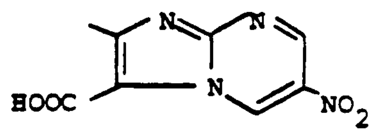 |
| **29** | n-$C_4H_9$ | CHOH | H | 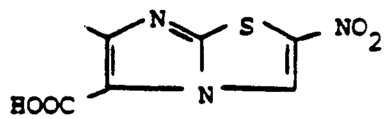 |
| 30 | n-$C_4H_9$ | $CH_2OH$ | H | 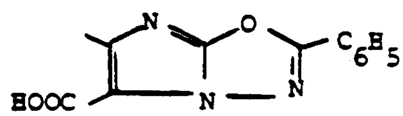 |
| 31 | n-$C_4H_9$ | $CH_2OH$ | H | 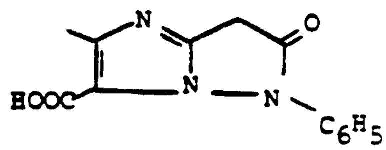 |
| 32 | n-$C_3H_7$ | CHO | H | 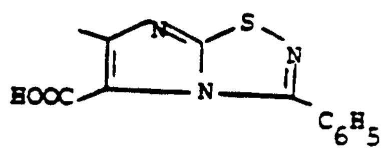 |
| 33 | n-$C_4H_9$ | CHO | H | 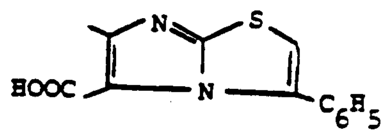 |
| 34 | n-$C_4H_9$ | $CH_2OH$ | H | 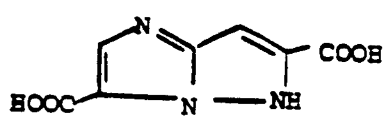 |

| | | | | |
|---|---|---|---|---|
| 35 | n-$C_4H_9$ | CHO | 2-$OCl_3$ | 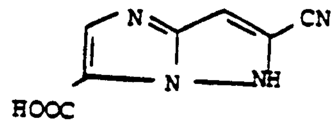 |
| 36 | n-$C_4H_9$ | $CH_2OH$ | H | 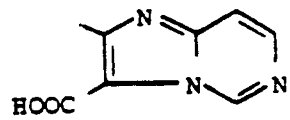 |
| 37 | n-$C_4H_9$ | CHO | H | 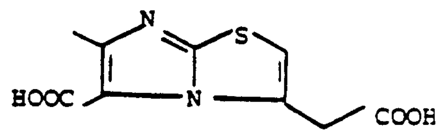 |
| 38 | n-$C_4H_9$ | $CH_2OH$ | H | 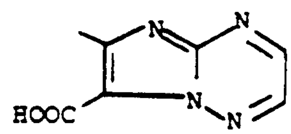 |
| 39 | n-$C_4H_9$ | CHO | H | 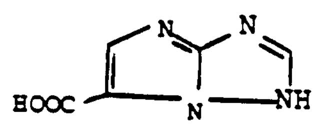 |
| 40 | n-$C_4H_9$ | $CH_2OH$ | H | 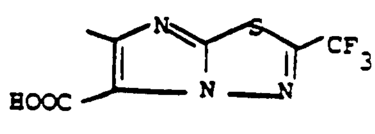 |

**Beispiel 41**

2-[4-[(2-n-Butyl-4-chlor-5-hydroxymethyl-imidazol-1-yl)methyl]phenyl]-3-(5-tetrazolyl)-imidazo[1,2-a]pyridin

a) α-Brom-2-tolyl-acetonitril

15,9 g (0,1 Mol) 3-p-Tolyl-3-oxo-propionitril (J. Amer. Chem. Soc. 69, 990 (1974) werden in 20 ml $CCl_4$ gelöst. Eine Lösung von 6 ml Brom in 30 ml $CCl_4$ wird bei -10 °C zugetropft. Nach 1 Std. bei -8 °C wird 3 Std. bei 20 °C, dann 1 Std. bei 60 °C gerührt. Das Lösungsmittel wird entfernt. Die Titelverbindung wird als Rohprodukt weiterverwendet.

b) 3-Cyano-2-(4-methyl-phenyl)-imidazo[1,2-a]pyridin

4,76 g (20 mmol) der Verbindung aus 41a) und 3,76 g (40 mmol) 2-Aminopyridin werden ohne Lösungsmittel 30 min auf 120 °C erhitzt. Nach Abkühlen wird an Kieselgel mit EtOAc/n-Heptan (1:3) chromatographiert. Man erhält 3,6 g Produkt als Öl. MS (DCI): 234 (M + H)

c) 2-(4-Brommethyl-phenyl)-3-cyano-imidazo[1,2-a]pyridin

2,34 g (10 mmol) der Verbindung aus 41b) werden zusammen mit 2 g NBS in 20 ml Chlorbenzol gelöst. Nach Zugabe von 200 mg Benzoylperoxid wird 90 min auf 120 °C erhitzt. Nach Abkühlen wird abgesaugt und das Filtrat 2x mit 1 N $NaHCO_3$-Lösung gewaschen. Die organische Phase wird über $Na_2SO_4$ getrocknet und eingeengt. Chromatographie an $SiO_2$ (EtOAc/n-Heptan 1:2) ergibt die Titelverbindung. MS (DCI): 312 + 314 (M + H)

d) 2-[4-[(2-n-Butyl-4-chlor-5-formyl-imidazol-1-yl)methyl]phenyl]-3-cyano-imidazo[1,2-a]pyridin

0,63 g (2 mmol) der Verbindung aus 41c), 0,37 g (2 mmol) 2-n-Butyl-4-chlorimidazol-5-aldehyd sowie 0,3 g $Na_2CO_3$ werden analog der in Beispiel 1d) angegebenen Vorschrift umgesetzt. Man erhält 0,7 g der Titelverbindung als Öl. MS (DCI): 418 (M + H)

e) 2-[4-(2-n-Butyl-4-chlor-5-hydroxymethyl-imidazol-1-yl)-methyl]phenyl]-3-cyano-imidazo[1,2-a]pyridin

0,22 g der Verbindung aus Beispiel 41d) werden analog der in Beispiel 2a) angegebenen Vorschritt mit 0,2 g $NaBH_4$ umgesetzt. Man erhält 0,2 g der Titelverbindung. MS (DCI): 420 (M + H)

f) 2-[4-(2-n-Butyl-4-chlor-5-hydroxymethyl-imidazol-1-yl)methyl]phenyl]-3-[1(3)-trimethylstannyl-tetrazol-5-yl]-imidazo[1,2-a]pyridin

0,2 g der Verbindung aus Beispiel 41 c) werden m 0,2 g Trimethylzinnazid in 5 ml Xylol 36 Std. auf 115 °C erhitzt ($N_2$). Nach Abkühlen wird abgesaugt und mit Toluol gewaschen. Man erhält 0,3 g der Titelverbindung, die als Rohprodukt weiter umgesetzt wird.

g) 2-[4-[2-n-Butyl-4-chlor-5-hydroxymethyl-imidazol-1-yl)methyl]phenyl]-3-[1(3)-triphenylmethyl-tetrazol-5-yl]-imidazo[1,2-a]pyridin

0,3 g der Verbindung aus 41f) werden in 5 ml $CH_2Cl_2$ und 1 ml Tetrahydrofuran mit 10 Äquivalenten 10 N NaOH versetzt. Nach 5 min werden 0,15 g Triphenylchlormethan zugegeben. Nach 24 Std. Rühren bei Raumtemperatur wird Wasser zugegeben, die organische Phase abgetrennt und eingeengt. Man erhält 0,27 g der Titelverbindung. MS (DCI): 703 (M + H)

h) 2-[4-(2-n-Butyl-4-chlor-5-hydroxymethyl-imidazol-1-yl)methyl]phenyl-3-(tetrazol-5-yl)-imidazo[1,2-a]-pyridin

0,27 g der Verbindung aus Beispiel 41f) werden in 3 ml Methanol mit 1 ml 5 N HCl versetzt. Nach 2 Std. bei Raumtemperatur wird mit Methanol verdünnt und mit 10 N NaOH auf PH 13 gestellt. Das Methanol wird im Vakuum entfernt. Der Rückstand wird mit Wasser verdünnt und mit Toluol 2x extrahiert. Die Wasserphase wird mit Eisessig neutralisiert und das Produkt abgesaugt. Man erhält 0,12 g der Titelverbindung. MS (DCI): 461 (M + H)

Die in der folgenden Tabelle aufgeführten Beispiele der Formel Ib wurden analog der in Beispiel 41 angegebenen Verfahren hergestellt.

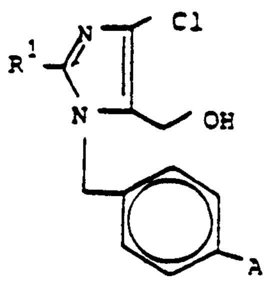

(Ib)

| Beispiel | $R^1$ | A |
|---|---|---|
| 42 | n-$C_4H_9$ | 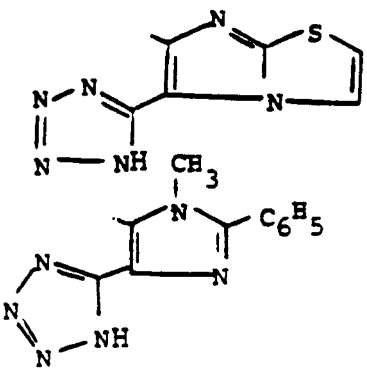 |
| 43 | n-$C_4H_9$ | |
| 44 | n-$C_5H_{11}$ | 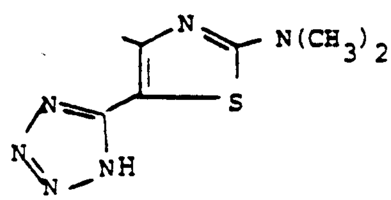 |
| 45 | n-$C_3H_7$ | 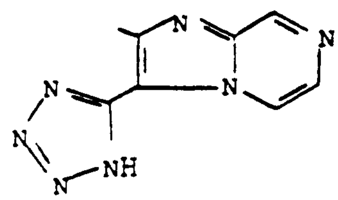 |
| 46 | n-$C_4H_9$ | 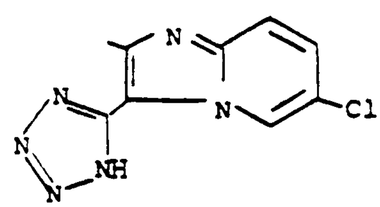 |
| 47 | n-$C_4H_9$ | 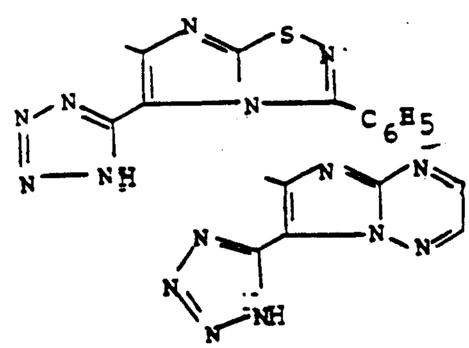 |
| 48 | n-$C_4H_9$ | |

| | | |
|---|---|---|
| 49 | n-$C_3H_7$ | 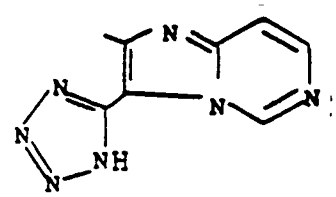 |
| 50 | n-$C_4H_9$ | 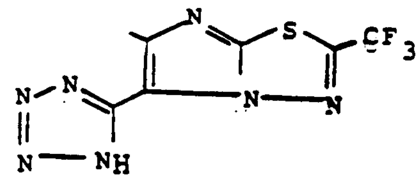 |
| 51 | n-$C_4H_9$ | 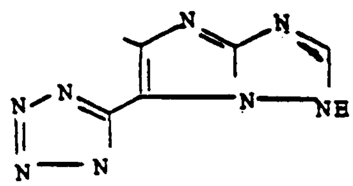 |

**Beispiel 52**

2-[4-[(3-Methoxymethyl-5-n-propyl-1,2,4-triazol-4-yl)methyl]phenyl]-imidazo[1,2-a]pyridin-4-carbonsäure

a) 2-[4-[(3-Methoxymethyl-5-n-propyl-1,2,4-triazol-4-yl)methyl]phenyl]-imidazo[1,2- a]pyridin-3-carbonsäure-ethylester
Hergestellt aus je 2 mmol der Verbindung aus Beispiel 1c) und 3-Methoxymethyl-5-n-propyl-1,2,4-triazol (bekannt aus EP-A-323 842) nach dem in Beispiel 1d) angegebenen Verfahren. MS (DCI): 434 (M + H)
b) 2-[4-[(3-Methoxymethyl-5-n-propyl-1,2,4-triazol-4-yl)methyl]phenyl]-imidazo[1,2- a]pyridin-3-carbonsäure
Hergestellt aus der Verbindung aus Beispiel 52a) nach dem in Beispiel 1e) angegebenen Verfahren.
MS (DCI): 406 (M + H)

**Beispiel 53**

2-[4-[(3-Methoxymethyl-5-n-butyl-pyrazol-1-yl)methyl]phenyl]-imidazo[1,2-a]pyridin-3-carbonsäure

a) 2-[4-[3-Methoxymethyl-5-n-butyl-pyrazol-1-yl)methyl]phenyl]-imidazo[1,2-a]pyridin-3-carbonsäure-ethylester
Hergestellt aus 1,2 mmol 3-Methoxymethyl-5-n-butyl-pyrazol (bekannt aus EP-A-323 842), 1,5 mmol der Verbindung aus Beispiel 1c) und 2 mmol Natriumhydrid bei 40 °C in DMF. Die Aufarbeitung erfolgte analog den in Beispiel 1d) angegebenen Verfahren. MS (DCI): 447 (M + H)
b) 2-[4-[(3-Methoxymethyl-5-n-butyl-pyrazol-1-yl)methyl]phenyl]imidazo[1,2-a]pyridin-3-carbonsäure
Hergestellt aus der Verbindung aus Beispiel 53a) nach den in Beispiel 1e) angegebenen Verfahren.
MS (DCI): 419 (M + H)

**Beispiel 54**

2-[4-[(2-n-Butyl-4-methylthio-5-carboxy-imidazol-1-yl)-methyl]phenyl]-imidazo[1,2-A]pyridin-3-carbonsäure

a) 2-Amino-2-cyano-essigsäureethylester
Zu 35 g (0,246 Mol) 2-Cyanoglyoxylsäureethylester-2-oxim in 350 ml $H_2O$ und 280 ml gesättigter Natriumhydrogencarbonat-Lösung werden bei Raumtemperatur portionsweise (15 min) 119 g Natriumdithionit zugegeben. Anschließend wird 1 Stunde auf 35 °C erwärmt; dann mit HaCl gesättigt und 5x mit Dichlormethan extrahiert. Nach Trocknen mit Calciumchlorid wird die organische Phase eingeengt. Man erhält 11,8 g der Titelverbindung als Öl. Rf ($CH_2Cl_2/CH_3OH$ 9/1) = 0,6
b) 2-Cyano-2-n-butylcarbonylaminoessigsäureethylester

Zu 3,6 g (28,09 mmol) Verbindung 2a) in 50 ml trockenem $CH_2Cl_2$ und 2,3 ml (28,09 mmol) Pyridin tropft man bei -5 °C bis 0 °C 3,39 ml (28,09 mmol) Valeroylchlorid in 5 ml $CH_2Cl_2$ zu. Anschließend wird 1 Stunde bei Raumtemperatur gerührt. Die organische Phase wird dann 3x mit $H_2O$ und 1x mit gesättigter NaCl-Lösung gewaschen, mit Calciumchlorid getrocknet und eingeengt.
Kristallisation aus DIP liefert 1,7 g der Titelverbindung.
$R_f$ ($CH_2Cl_2$/$CH_3OH$ 9/1) = 0,35
Schmp.: 87 °C

c) 3-Amino-2-n-butylcarbonylamino-methylthioacrylsäureethylester

Zu 2,9 g (13,67 mmol) Verbindung 2b) und 0,19 ml (1,36 mmol) Triethylamin in 60 ml absolutem Ethanol gibt man bei Raumtemperatur 2 ml (27,26 mmol) kondensiertes Methylmercaptan. Mach 3 Tagen gibt man nachmals 0,5 ml Methylmercaptan zu. Nach weiteren 24 Stunden bei Raumtemperatur werden nochmals 0,5 ml Methylmercaptan und 0,19 ml Triethylamin in zugespritzt und weitere 24 Stunden bei Raumtemperatur gerührt. Anschließend wird das Lösungsmittel entfernt und der Rückstand aus DIP kristallisiert, wobei 2,4 g der Titelverbindung anfallen.
$R_f$ ($CH_2Cl_2$/EE 4/1) = 0,3
Schmp.: 120 °C

d) 2-n-Butyl-4-methylthio-imidazol-5-carbonsäureethylester

Zu 4,17 g (20,0 mmol) Phosphorpentachlorid in 20 ml $CH_2Cl_2$ tropft man bei -78 °C 2,44 g (20,0 mmol) 4-Dimethylaminopyridin in 12 ml $CH_2Cl_2$. Nach 5 min werden 2,42 g (10,0 mmol) Verbindung 2c in 25 ml $CH_2Cl_2$ zugetropft. Nun läßt man auf Raumtemperatur kommen und verdünnt mit 30 ml $CH_2Cl_2$. Nach 2 Stunden werden unter Eiskühlung 300 ml 1n Natriumhydrogencarbonat-Lösung zugegeben und 1 Stunde gerührt. Nun werden die Phasen getrennt, die wäßrige Phase 3x mit EE extrahiert und die vereinigten organischen Phasen mit Calciumchlorid getrocknet. Chromatographie an $SiO_2$ mit $CH_2Cl_2$/EE (9/1) $R_f$ - ($CH_2Cl_2$/EE 9/1) = 0,6 MS (DCI) = 243 ($M^+$ +H)

e) 2-[4-[(2-n-Butyl-4-methylthio-5-ethoxycarbonyl-imidazol-1-yl)-methyl]-phenyl]-imidazo-[1,2-A]pyridin-3-carbonsäureethylester

0,71 g (1,97 mmol) der Verbindung aus Beispiel 1c), 0,48 g (1,97 mmol) der Verbindung aus Beispiel 54d) und 0,90 g (6,48 mmol) Kaliumcarbonat werden in 10 ml abs. DMF 24 h bei Raumtemperatur gerührt. Die Reaktionslösung wird zur Trockene eingeengt, der Rückstand in EE gelöst, die EE-Lösung 3x mit $H_2O$ und 1x mit ges. $NaHCO_3$-Lösung gewaschen, über $Na_2SO_4$ getrocknet und eingeengt. Chromatographie an Kieselgel mit EE/Heptan 1/1 und 4/1 lieferte 0,51 g der Titelverbindung als Öl.
$R_f$ ($SiO_2$, EE/Heptan 4/1) = 0,4
MS (FAB): 521 (M + H)

f) 2-[4-[(2-n-Butyl-4-methylthio-5-carboxy-imidazol-1-yl)-methyl]-phenyl]-imidazo[1,2-A]pyridin-3-carbonsäure

0,2 g (0,395 mmol) der Verbindung aus Beispiel 54e) wurde in 5 ml Ethanol mit 4 ml 1N NaOH 5d bei RT gerührt. Die Reaktionslösung wurde mit $H_2O$ verdünnt, mit 2n $H_2SO_4$ auf pH = 3 eingestellt und mit EE extrahiert. Der beim Einengen der EE-Lösung ausfallende Niederschlag wurde abgesaugt und im Hochvakuum getrocknet. Es resultierten 60 mg der Titelverbindung.
m.p. = 199 °C (Zersetzung)
MS (FAB): 493 (M + H)

**Beispiel 55**

2-[4-[(2-n-Butyl-4-methylsulfinyl-5-carboxy-imidazol-1-yl)-methyl]-phenyl]imidazol[1,2-A]pyridin-3-carbonsäure

a) 2-[4-[2-n-Butyl-4-methylsulfinyl-5-ethoxycarbonyl-imidazo-1-yl)-methyl]-phenyl]-imidazo [1,2-A]-pyridin-3-carbonsäureethylester

300 mg (0,577 mmol) der Verbindung aus Beispiel 54e) wurden in 10 ml abs. $CH_2Cl_2$ mit 0,199 g (0,577 mmol) 3-Chlorperoxybenzoesäure (50 %ige Lösung) 3 h bei Raumtemperatur gerührt. Es wurde mit 10 %iger Natriumbisulfit-Lösung versetzt, mit EE extrahiert, die vereinigten organ. Phasen mit 10 %iger $Na_2CO_3$-Lösung gewaschen, über $Na_2SO_4$ getrocknet und eingeengt. Chromatographie an Kieselgel liefert 250 mg der Titelverbindung. MS (FAB): 537 (M + H)

b) 2-[4-[2-n-Butyl-4-methylsulfinyl-5-carboxy-imidazol-1-yl)methyl]-phenyl]-imidazo[1,2-A]pyridin-3-carbonsäure

250 mg (0,466 mmol) der Verbindung aus Beispiel 55a) wurde nach dem in Beispiel 54f) angeführten Verfahren zur Titelverbindung umgesetzt. Es resultierten 50 mg.

MS (FAB): 481 (M + H)

**Beispiel 56**

2-[4-[2n-Butyl-4-methylsulfonyl-5-carboxy-imidazol-1-yl)methyl]-phenyl]-imidazo[1,2-A]pyridin-3-carbonsäure

a) 2-[4-[(2n-Butyl-4-methylsulfonyl-5-carboxy-imidazol-1-yl)methyl]-phenyl]-imidazo[1,2-A]pyridin-3-carbonsäureethylester

200 mg (0,385 mmol) der Verbindung aus Beispiel 54e) wurden in 10 ml abs. $CH_2Cl_2$ mit 0,266 g (0,77 mmol) 3-Chlorperoxybenzoesäure (50 %ig) 15 h am Rückfluß gekocht. Die Reaktionslösung wurde mit 10 %iger Natriumbisulfit-Lösung versetzt, mit EE extrahiert, die vereinigten organ. Phasen mit 10 %iger $Na_2CO_3$-Lösung gewaschen, über $Na_2SO_4$ getrocknet und eingeengt. Chromatographie an Kieselgel mit EE/Heptan (4:1) lieferte 130 mg der Titelverbindung.

MS (FAB): 553 (M + H)

b) 2-[4-[2-n-Butyl-4-methylsulfonyl-5-carboxy-imidazol-1-yl)methyl]-phenyl]-imidazo[1,2-A]pyridin-3-carbonsäure

Die Titelverbindung wurde aus der Verbindung aus Beispiel 56a) nach dem in Beispiel 54f) angeführten Verfahren hergestellt.

MS (FAB): 497 (M + H)

**Beispiel 57**

2-[4-[(2-n-Butyl-4-methylthio-5-carboxy-imidazol-1-yl)methyl]-phenyl]-3-(1H-5-tetrazolyl)-imidazo[1,2-A]-pyridin

a) 2-[4-[(2-n-Butyl-4-methylthio-5-ethoxycarbonyl-imidazol-1-yl)-methyl]phenyl]-3-cyano imidazo[1,2-A]-pyridin

1,09 g (3,5 mmol) der Verbindung aus Beispiel 41c), 0,85 g (3,5 mmol) der Verbindung aus Beispiel 54d) und 1,45 g (10,5 mmol) $K_2CO_3$ werden analog der in Beispiel 54e) angegebenen Vorschrift umgesetzt. Man erhält 1,0 g der Titelverbindung als schwach beigegefärbten Feststoff.

m.p. = 168 °C

MS (FAB): 474 (M + H)

b) 2-[4-[(2-n-Butyl-4-methylthio-5-ethoxycarbonyl-imidazol-1-yl)methyl]phenyl]-3-(1H-tetrazol-5-yl)-imidazo[1,2-A]pyridin

473 mg (1mmol) der Verbindung aus Beispiel 57a) werden mit 310 mg (1,5 mmol) Trimethylzinnazid in 3 ml Toluol 3h zum Rückfluß erhitzt. Die Reaktionslösung wurde mit 2 ml Diethylether verdünnt und mit 20 ml gesättigte KF-Lösung und 0,2 ml $HBF_4$-Lösung (50 %ig) 16 h bei Raumtemperatur gerührt. Es wurde mit EE verdünnt, filtriert, die EE-Phase abgetrennt und über $Na_2SO_4$ getrocknet. Einengung der EE-Phase und Chromatographie an Kieselgel mit EE/Methanol (3:1) lieferte 34,0 mg der Titelverbindung.

m.p.: 180-215 °C

MS (FAB): 517 (M + H)

c) 2-[4-[(2-n-Butyl-4-methylthio-5-carboxy-imidazol-1-yl)methyl]-phenyl]-3-(1H-tetrazol-5-yl)-imidazo-[1,2-A]-pyridin

180 mg (0,35 mmol) der Verbindung aus Beispiel 57b) wurde nach dem in Beispiel 54f) angeführten Verfahren umgesetzt. Nach einer Reaktionszeit von 5h resultierten 55mg der Titelverbindung.

m.p.: 160 °C

MS (FAB): 489 (M + H)

Die in der folgenden Tabelle aufgelisteten Beispiele der Formel Ic wurden analog der in den Beispielen 54-57 angegebenen Verfahren hergestellt.

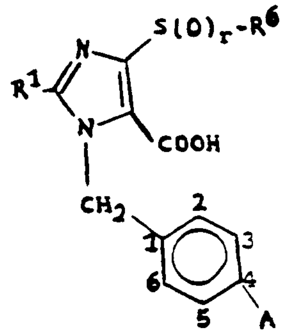

(Ic)

| Beispiel | $R^1$ | r | $R^6$ | A | FAB-MS (M+H) |
|---|---|---|---|---|---|
| 58 | n-$C_3H_7$ | O | $CH_3$ | 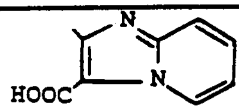 | 451 |
| 59 | n-$C_3H_7$ | 2 | $CH_3$ | 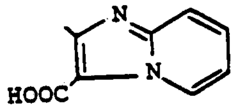 | 483 |
| 60 | n-$C_4H_9$ | 0 | $C_2H_5$ | 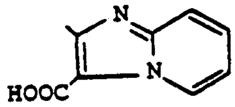 | 479 |
| 61 | n-$C_4H_9$ | 1 | $C_2H_5$ | 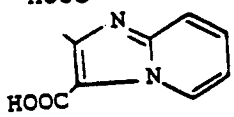 | 495 |
| 62 | n-$C_4H_9$ | 2 | $C_2H_5$ | 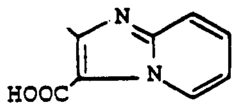 | 511 |
| 63 | n-$C_4H_9$ | 0 | $CH_3$ | 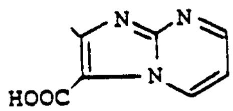 | 466 |
| 64 | n-$C_4H_9$ | 0 | $CH_3$ | 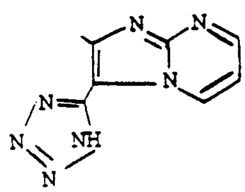 | 490 |
| 65 | n-$C_4H_9$ | 0 | $CH_3$ | 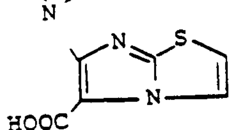 | 471 |
| 66 | n-$C_4H_9$ | 0 | $CH_3$ | 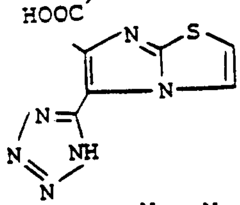 | 495 |
| 67 | n-$C_4H_9$ | 0 | $CH_3$ | 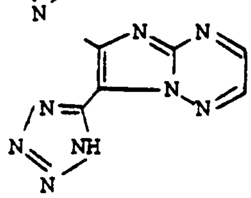 | 491 |
| 68 | n-$C_4H_9$ | 0 | $CH_3$ | 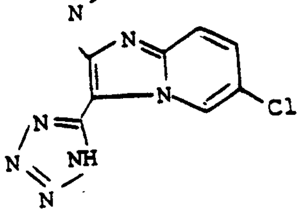 | 523 |

## Patentansprüche

**1.** Verbindung der Formel (I)

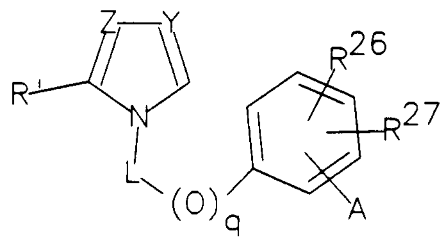

(I)

in welcher

a) X, Y und Z gleich oder verschieden sind und N oder $CR^2$ bedeuten,

b)

$R^1$

1. $(C_2-C_{10})$-Alkyl,
2. $(C_3-C_{10})$-Alkenyl,
3. $(C_3-C_{10})$-Alkinyl,
4. $OR^3$,
5. $(C_3-C_8)$-Cycloalkyl,
6. $(C_4-C_{10})$-Cycloalkylalkyl,
7. $(C_5-C_{10})$-Cycloalkylalkenyl,
8. $(C_5-C_{10})$-Cycloalkylalkinyl,
9. $-(CH_2)_m-B-(CH_2)n-R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1-C_4)$-Alkoxy und Nitro substituiert ist,

bedeutet;

c)

$R^2$

1. Wasserstoff,
2. Halogen,
3. Nitro,
4. $C_vF_{2v+1}$,
5. Pentafluorphenyl,
6. Cyano,
7. Phenyl,
8. Phenyl-$(C_1-C_3)$-alkyl,
9. $(C_1-C_{10})$-Alkyl,
10. $(C_3-C_{10})$-Alkenyl,
11. Phenyl-$(C_2-C_6)$-Alkenyl,
12. 1-Imidazolyl-$(CH_2)_m$-,
13. 1,2,3-Triazolyl-$(CH_2)_n$-,
14. Tetrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,
21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,
25.

$$-(CH_2)_o-O-\underset{\underset{W}{\|}}{C}-NH-R^9,$$

26.

$$-(CH_2)_o-NR^7-\underset{\underset{W}{\|}}{C}-OR^9,$$

27. $-(CH_2)_o-NR^7-CO-NHR^9$,
28. $-(CH_2)_o-NR^7-SO_2R^9$,
29.

$$-(CH_2)_o-NR^7-\underset{\underset{W}{\|}}{C}-R^9,$$

30. $-(CH_2)_nF$,
31. $-(CH_2)_n-O-NO_2$,
32. $-CH_2-N_3$,
33. $-(CH_2)_n-NO_2$,
34. $-CH=N-NR^5R^7$,
35. Phthalimido-$(CH_2)_n$-,
36.

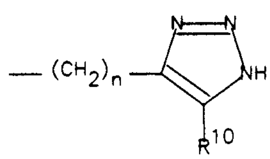

37.

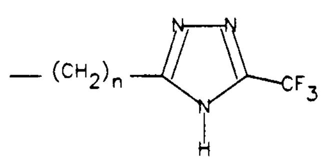

38.

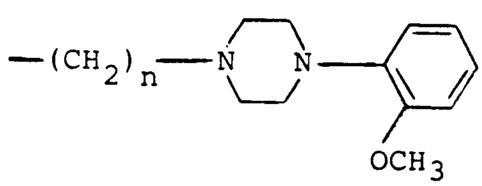

39.

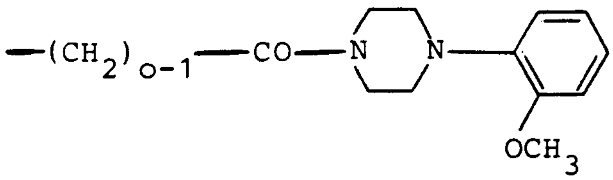

40. Phenyl-$SO_2$-NH-N = CH-,

41.

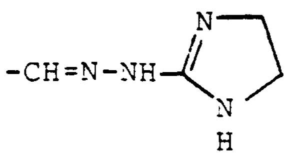

42. -$(CH_2)_n$-$SO_2$-$NR^7$-CO-$NR^6R^9$,

43. -$(CH_2)_o$-$SO_2R^9$,

44. einem wie unter c) 9. oder 10. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,

45. einem wie unter c) 9. oder 10. definierten Rest, worin 1 H-Atom durch Hydroxy oder worin 1 bis alle H-Atome durch Fluor substituiert sind, oder

46. den unter c) 13. definierten Rest, der mit 1 oder 2 gleichen oder verschiedenen Resten aus der Reihe Methoxycarbonyl und ($C_1$-$C_4$)-Alkyl substituiert ist,

bedeutet;

d)

$R^3$
1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,

bedeutet;

e)

$R^4$
1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl

bedeutet;

f)

$R^5$
1. Wasserstoff,
2. ($C_1$-$C_6$)-Alkyl,
3. ($C_3$-$C_8$)-Cycloalkyl,
4. Phenyl oder
5. Benzyl

bedeutet;

g)

$R^6$
1. Wasserstoff,
2. ($C_1$-$C_6$)-Alkyl,
3. ($C_3$-$C_8$)-Cycloalkyl,
4. ($C_6$-$C_{12}$)-Aryl, vorzugsweise Phenyl,
5. Benzyl,
6. ($C_1$-$C_9$)-Heteroaryl, vorzugsweise 2-Pyrimidinyl,

7. $(C_1-C_4)$-Alkanoyl,
8. einen wie unter g) 4. oder 6. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl,

bedeutet;

h)
$R^7$
1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl, vorzugsweise Benzyl,
5. Phenyl oder
6. $(C_1-C_9)$-Heteroaryl

bedeutet;

i)
$R^8$
1. Wasserstoff,
2. $(C_1-C_6)$-Alkyl,
3. $(C_3-C_8)$-Cycloalkyl,
4. Phenyl-$(CH_2)_q$-,
5. $OR^5$,
6. $NR^{11}R^{12}$ oder
7.

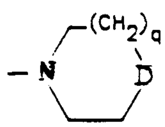

bedeutet;

j)
$R^9$
1. $(C_1-C_6)$-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,

bedeutet;

k)
$R^{10}$ Cyano, Nitro oder $CO_2R^7$ bedeutet;

l)
$R^{11}$ und $R^{12}$ gleich oder verschieden sind und
1. Wasserstoff,
2. $(C_1-C_4)$-Alkyl,
3. Phenyl,
4. Benzyl oder
5. $\alpha$-Methylbenzyl

bedeuten;

m)
D $NR^{13}$, O oder $CH_2$ bedeutet;

n)
$R^{13}$ Wasserstoff, $(C_1-C_4)$-Alkyl oder Phenyl bedeutet;

o)
A den Rest eines Heterocyclus mit 5-10 Ringatomen, der mono- oder bicyclisch sein kann, und wovon bis zu 9 Ringatome C-Atome sind, der mit bis zu 6, vorzugsweise bis zu 3 gleichen oder verschiedenen Resten $R^{14}$ oder $-(CH_2)_{n-1}-(CHR^6-CH_2)_{o-1}-R^{15}$ substituiert sein kann, und der ungesättigt oder teilhydriert sein kann, bedeutet;

p)
$R^{14}$ 1. Halogen,

2. Oxo,
3. Nitroso,
4. Nitro, 5. Amino,
6. Cyano,
7. Hydroxy,
8. $(C_1-C_6)$-Alkyl,
9. $(C_1-C_4)$-Alkanoyl,
10. $(C_1-C_4)$-Alkanoyloxy,
11. $CO_2R^3$,
12. Methansulfonylamino,
13. Trifluormethansulfonylamino,
14. $-CO-NH-OR^9$,
15. $-SO_2-NR^6R^7$,
16. $-CH_2-OR^7$,
17. $(C_1-C_9)$-Heteroaryl-$(CH_2)_q$-, vorzugsweise 1-Tetrazolyl,
18. $(C_7-C_{13})$-Aroyl,
19.

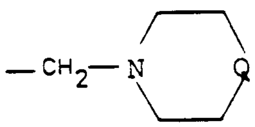

20.

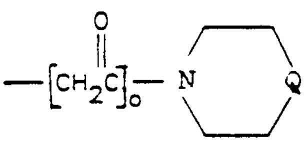

21. $(C_6-C_{12})$-Aryl
bedeutet;

q)
$R^{15}$
1. Wasserstoff,
2. $(C_1-C_6)$-Alkyl,
3. $(C_3-C_8)$-Cycloalkyl,
4. $(C_6-C_{12})$-Aryl,
5. $(C_7-C_{13})$-Aroyl,
6. $(C_1-C_4)$-Alkoxy,
7. $(C_1-C_4)$-Alkanoyloxy,
8. $(C_1-C_9)$-Heteroaryl,
9. $CO_2R^3$,
10. Halogen,
11. Cyano,
12. Nitro,
13. $NR^6R^7$,
14. Hydroxy,
15. $-CO-NH-CHR^5-CO_2R^3$,
16. Sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. Phosphonooxy,
22. $-PO_3H_2$,

23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. den unter p) 20. definierten Rest,
29.

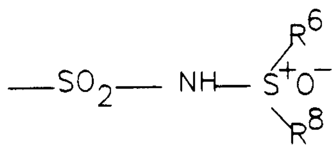

30.

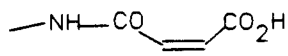

31.

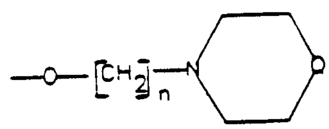

32. 5-Tetrazolyl-NH-CO-,
33. $-CO-NH-NH-SO_2CF_3$,
34.

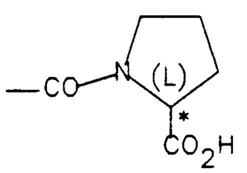

35.

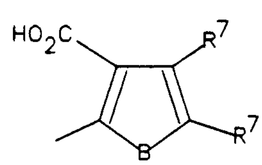

36.

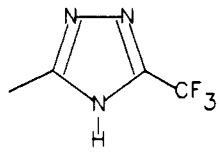

37.

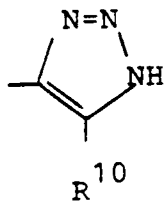

38.

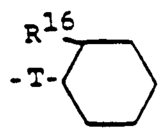

39.

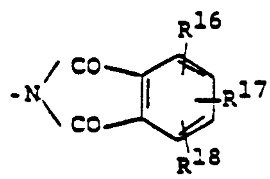

40.

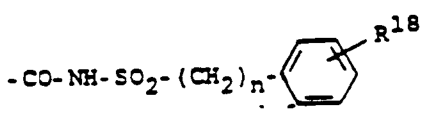 oder

41. den unter q) 4. definierten Rest, substituiert mit 1 oder 2 gleichen oder verschiedenen Resten aus der Reihe Halogen, Cyano, Nitro, $NR^6R^7$ und Hydroxy bedeutet;

r)
B O, $NR^7$ oder S bedeutet;

s)
W O oder S bedeutet;

t)
L $(C_1-C_3)$-Alkandiyl bedeutet;

u)
$R^{16}$ $CO_2R^3$ oder $CH_2CO_2R^3$ bedeutet;

v)
$R^{17}$ Wasserstoff, Halogen, $(C_1-C_4)$-Alkyl oder $(C_1-C_4)$-Alkoxy bedeutet;

w)
$R^{18}$ Wasserstoff, $(C_1-C_4)$-Alkyl oder Phenyl bedeutet;

x)
$R^{19}$ 1. Wasserstoff,

2. Hydroxy,
3. ($C_1$-$C_6$)-Alkanoyloxy,
4. ($C_1$-$C_6$)-Alkyl,
5. ($C_2$-$C_6$)-Alkenyl,
6. ($C_3$-$C_8$)-Cycloalkyl,
7. Phenyl,
8. Benzyl oder
9. Phenacyl

bedeutet;

y)

T
1. eine Einfachbindung,
2. -CO-,
3. $-CH_2-$,
4. -O-,
5. -S-,
6. $NR^{21}-$,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,
15. $SO_2-NR^{21}-$,
16. $-CR^{20}R^{22}-NH-$,
17. -CH = CH-,
18. -CF = CF-,
19. -CH = CF-,
20. -CF = CH-,
21. $-CH_2-CH_2-$,
22. $-CF_2-CF_2-$,
23. $-CH(OR^3)-$,
24. $-(CH(OCOR^5)-$,
25.

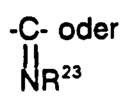 oder

26.

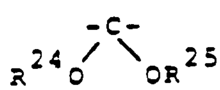

bedeutet;

z)

$R^{20}$ und $R^{22}$ gleich oder verschieden sind und Wasserstoff, ($C_1$-$C_5$)-Alkyl, Phenyl, Allyl oder Benzyl bedeutet;

a')

$R^{21}$ Wasserstoff, ($C_1$-$C_6$)-Alkyl, Benzyl oder Allyl bedeutet;

b')

$R^{23}$
1. $NR^{20}R^{21}$,
2. Ureido,
3. Thioureido,

4. Toluol-4-sulfonyl oder
5. Benzolsulfonylamino

bedeuten;

c')

$R^{24}$ und $R^{25}$ gleich oder verschieden sind und ($C_1$-$C_4$)-Alkyl bedeuten oder gemeinsam für -($CH_2$)$_q$- stehen;

d')

$R^{26}$ und $R^{27}$ gleich oder verschieden sind und
1. Wasserstoff,
2. Halogen,
3. Nitro,
4. ($C_1$-$C_4$)-Alkyl oder
5. ($C_1$-$C_2$)-Alkoxy

bedeuten;

e')

Q $CH_2$, NH, O oder S bedeutet;

f')

m eine ganze Zahl von 0 bis 5 ist;

g')

n eine ganze Zahl von 1 bis 5 ist;

h')

o eine ganze Zahl von 1 bis 10 ist;

i')

q 0 oder 1 ist;

j')

r 0, 1 oder 2 ist, oder

k')

v eine ganze Zahl von 1 bis 6 ist;

sowie deren physiologisch verträglichen Salze.

**2.** Verbindung der Formel (I) gemäß Anspruch 1, in welcher

a) X N, Y $CR^2$ und Z $CR^2$ bedeuten;
b) X $CR^2$, Y N und Z $CR^2$ bedeuten;
c) X $CR^2$, Y $CR^2$ und Z N bedeuten
oder
d) X, Y und Z jeweils N bedeuten
sowie deren physiologisch verträglichen Salze.

**3.** Verbindung der Formel (I) gemäß Anspruch 1, in welcher

a)

$R^1$
1. ($C_3$-$C_{10}$)-Alkyl,
2. ($C_3$-$C_{10}$)-Alkenyl,
3. ($C_3$-$C_{10}$)-Alkinyl,
4. ($C_3$-$C_8$)-Cycloalkyl,
5. Benzyl oder
6. Benzyl, das wie im Anspruch 1 definiert substituiert ist, bedeutet;

b)

$R^2$
1. Wasserstoff,
2. Halogen,
3. Nitro,
4. $C_vF_{2v+1}$,
5. Pentafluorphenyl,
6. Cyano,
7. Phenyl,
8. Phenyl-($C_1$-$C_3$)-alkyl,
9. ($C_1$-$C_{10}$)-Alkyl,

10. ($C_3$-$C_{10}$)-Alkenyl,
11. Phenyl-($C_2$-$C_6$)-alkenyl,
12. 1-Imidazolyl-$(CH_2)_m$-,
13. 1,2,3-Triazolyl-$(CH_2)_o$-,
14. Tetrazolyl-$(CH_2)_m$-,
15. -$(CH_2)_{0-1}$-$CHR^7$-$OR^5$,
16. -$(CH_2)_o$-O-$COR^3$,
17. -$COR^8$,
18. -$(CH_2)_o$-CO-$R^8$,
19. -$S(O)_rR^6$,
20. -CH = CH-$(CH_2)_m$-$CHR^3$-$OR^6$,
21. -$CH_2$ = CH-$(CH_2)_m$-CO-$R^8$,
22. -$(CH_2)_o$-NH-CO-$OR^9$,
23. -$(CH_2)_o$-NH-$SO_2$-$R^9$,
24. -$(CH_2)_nF$,
25. -$(CH_2)_o$-$SO_3R^9$,
26. -$(CH_2)_n$-$SO_2$-NH-CO-$NR^6R^9$ oder
27 einem wie unter b) 7., 8., 9., 10. oder 15. definierten Rest, der, wie in Anspruch 1 unter c) 44., 45. oder 46. jeweils für einen solchen Rest beschrieben substituiert ist,

bedeutet;

c)

$R^8$ Wasserstoff; ($C_1$-$C_5$)-Alkyl, $OR^5$, $NR^{11}R^{12}$ oder Morpholino bedeutet;

d)

T 1. eine Einfachbindung, 2. -CO-,
3. -$CONR^{21}$-,
4. -$CH_2$-$CH_2$-,
5. -$NR^{21}$-CO-,
6. -O-$CH_2$-,
7. -$CH_2$-O-,
8. -S-$CH_2$-,
9. -$CH_2$-S-,
10. -NH-$CH_2$-,
11. -$CH_2$-NH- oder
12. -CH = CH-

bedeutet,

sowie deren physiologisch verträglichen Salze.

**4.** Verbindung der Formel (I) gemäß Anspruch 1, in welcher

a)

$R^1$ ($C_3$-$C_7$)-Alkyl, ($C_3$-$C_7$)-Alkenyl oder ($C_3$-$C_7$)-Alkinyl bedeutet;

b)

$R^2$ 1. Chlor,
2. Brom,
3. $C_v F_{2v+1}$ mit v = 1, 2 oder 3,
4. Pentafluorphenyl,
5. -$S(O)_rR^6$,
6. $(CH_2)_{0-1}$-$CHR^7OR^5$,
7. $(CH_2)_o$-O-CO-$R^3$,
8. -$COR^8$,
9. -$(CH_2)_o$-CO-$R^8$,
10. -$CH_2$-NH-CO-$R^8$,
11. -$(CH_2)_o$-NH-$SO_2$-$R^9$,
12. -CH = CH-$CHR^3$-$OR^6$,
13. Tetrazolyl-$(CH_2)_m$-,
14. -$(CH_2)_nSO_2$-NH-CO-$NR^6R^9$,
15. -$(CH_2)_o$-$SO_3R^9$ oder

gegebenenfalls durch Hydroxy substituiertes ($C_1$-$C_6$)-Alkyl, vorzugsweise Hydroxymethyl bedeutet;

c)

$R^3$ Wasserstoff oder ($C_1$-$C_4$)-Alkyl bedeutet;

d)

$R^6$ Wasserstoff, ($C_1$-$C_4$)-Alkyl, ($C_1$-$C_4$)-Alkanoyl oder vorzugsweise ($C_2$-$C_9$)-Heteroaryl bedeutet;

e)

$R^7$ Wasserstoff, ($C_1$-$C_4$)-Alkyl, ($C_1$-$C_9$)-Heteroaryl, oder ($C_6$-$C_{12}$)-Aryl-($C_1$-$C_4$)-Alkyl bedeutet;

f)

$R^8$ Wasserstoff, ($C_1$-$C_4$)-Alkyl, $OR^5$ oder Morpholino bedeutet;

g)

$R^9$ $CF_3$, ($C_1$-$C_6$)-Alkyl oder Phenyl bedeutet;

h)

$R^{14}$

1. ($C_1$-$C_4$)-Alkyl,
2. ($C_1$-$C_4$)-Alkoxy,
3. Cyano,
4. Amino,
5. Nitroso,
6. Nitro,
7. Fluor,
8. Chlor,
9. Brom,
10. Hydroxy,
11. $CH_2OR^7$,
12. ($C_1$-$C_9$)-Heteroaryl-$CH_2$-,
13. ($C_1$-$C_4$)-Alkanoyloxy,
14. ($C_1$-$C_4$)-Alkanoyl,
15. Benzoyl,
16.

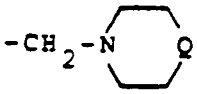

17. -NH-CO-$R^7$ oder
18. Tetrazolyl

bedeutet;

i)

$R^{15}$

1. ($C_1$-$C_4$)-Alkyl,
2. ($C_6$-$C_{12}$)-Aryl,
3. ($C_1$-$C_3$)-Alkanoyloxy,
4. ($C_1$-$C_4$)-Alkoxy,
5. ($C_1$-$C_9$)-Heteroaryl, vorzugsweise 5-Tetrazolyl,
6. Cyano,
7. Nitro,
8. Hydroxy,
9. -$S(O)_rR^6$,
10. -$SO_3R^3$,
11. Chlor,
12. Brom,
13. Benzoyl,
14. -$CO_2R^3$,
15. -CO-NH-$R^6$,
16. -$NR^6R^7$,
17. -CO-$R^8$,
18. -$SO_2$-$NR^6R^7$,
19.

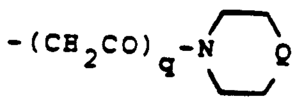

20.

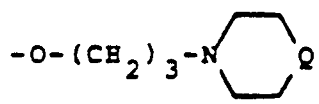

21. $-SO_2-NH-CO-NR^6R^9$,
22. $-PO_3H_2$,
23. $-CO-CHR^5-CO_2H$,
24. $-NH-CO-NH-SO_2-CH_2-R^5$,
25. 5-Tetrazolyl-NH-CO-,
26.

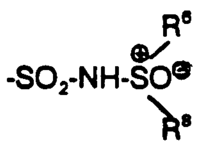

27.

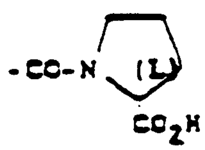

28.

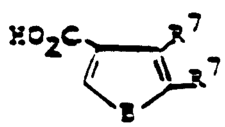

29.

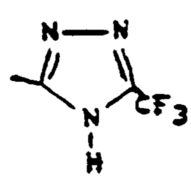

30.

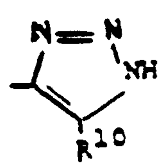

31.

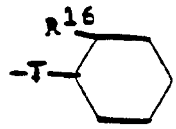

32.

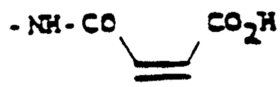 ,

33.

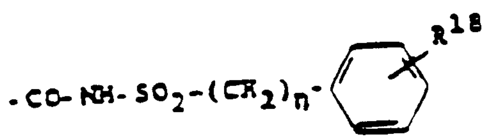 oder

34. den unter i) 2. definierten Rest, substituiert wie oben definiert, bedeutet;

j)

Q $CH_2$, NH oder O bedeutet;

k)

$R^{18}$ Wasserstoff, Methyl oder Ethyl bedeutet;

l)

T eine Einfachbindung, -O-, -CO-, -NHCO- oder $-OCH_2-$ bedeutet,

sowie deren physiologisch verträglichen Salze.

**5.** Verfahren zur Herstellung von Verbindungen der Formel I gemäß Anspruch 1, dadurch gekennzeichnet, daß man Verbindungen der Formel (II)

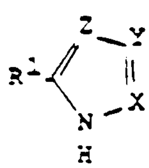

(II)

worin $R^1$, X, Y und Z wie oben definiert sind, alkyliert mit Verbindungen der Formel III

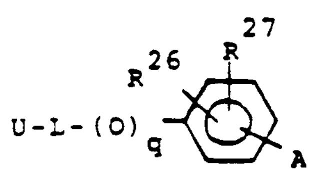

(III)

worin L, A und q wie oben definiert sind, und U für eine Fluchtgruppe steht, gegebenenfalls temporär eingeführte Schutzgruppen wieder abspaltet und die erhaltenen Verbindungen der Formel (I) gegebenenfalls in ihre physiologisch verträglichen Salze überführt.

**6.** Verbindung gemäß Anspruch 1 zur Anwendung als Heilmittel.

7. Verbindung gemäß Anspruch 1 zur Anwendung als Heilmittel bei der Behandlung des Bluthochdrucks.

8. Pharmazeutische Zubereitung, enthaltend mindestens eine Verbindung gemäß Anspruch 1.

9. Verfahren zur Herstellung einer Zubereitung gemäß Anspruch 8, dadurch gekennzeichnet, daß man den oder die Wirkstoffe zusammen mit einem physiologisch unbedenklichen Träger und gegebenenfalls weiteren Zusatz- und Hilfsstoffen in eine geeignete Darreichungsform bringt.